(19) Europäisches Patentamt
European Patent Office
Office européen des brevets

(11) **EP 4 756 417 A1**

(12) **EUROPEAN PATENT APPLICATION**

(43) Date of publication:
**10.06.2026   Bulletin 2026/24**

(21) Application number: **25202136.5**

(22) Date of filing: **15.09.2025**

(51) International Patent Classification (IPC):
***G01N 23/046*** (2018.01)

(52) Cooperative Patent Classification (CPC):
**G01N 23/046; A61B 6/032; A61B 6/035; A61B 6/4208; A61B 6/4275; G01V 5/226;** A61B 6/4007; A61B 6/4266; G01N 2223/419; G01N 2223/501

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority:   **04.12.2024   CN 202411766743**

(71) Applicants:
• **Tsinghua University**
  **Haidian District,**
  **Beijing 100084 (CN)**
• **Nuctech Company Limited**
  **TongFang Building**
  **Shuangqinglu**
  **Haidian District**
  **Beijing 100084 (CN)**

(72) Inventors:
• **CHEN, Zhiqiang**
  **Beijing 100084 (CN)**
• **ZHANG, Li**
  **Beijing 100084 (CN)**
• **YANG, Hongkai**
  **Beijing 100084 (CN)**
• **CHEN, Changyu**
  **Beijing 100084 (CN)**
• **HUANG, Qingping**
  **Beijing 100084 (CN)**
• **SHEN, Le**
  **Beijing 100084 (CN)**

(74) Representative: **Isarpatent**
**Patent- und Rechtsanwälte**
**Barth Hassa Peckmann & Partner mbB**
**Friedrichstraße 31**
**80801 München (DE)**

(54) **CT IMAGING SYSTEM**

(57)     Embodiments of the present disclosure provide a CT imaging system, which relates to the field of ray scanning technologies. The CT imaging system includes: an imaging channel for placing an object to be imaged in an imaging process; M distributed X-ray sources arranged at intervals along a circumferential direction of the imaging channel, at least one distributed X-ray source including q target spots; N detectors arranged at intervals along the circumferential direction of the imaging channel, including a first sub-detector and a second sub-detector. The CT imaging system includes a plurality of imaging components, the imaging component includes at least one distributed X-ray source and at least one first sub-detector. In the same imaging component, the distributed X-ray source and the first sub-detector are arranged face-to-face in a radial direction of the imaging channel, target spots of the distributed X-ray source and at least a part of the first sub-detector are located in a same plane perpendicular to the first direction. At least one second sub-detector is provided on at least one side of at least one distributed X-ray source in the first direction.

FIG. 6

## Description

TECHNICAL FIELD

The present disclosure relates to a field of ray scanning technologies, and in particular, to a CT imaging system.

BACKGROUND

A CT (computed tomography) scanning system relates to a nondestructive detecting technology widely used in fields such as biomedical, image-guided intervention, security inspection, industrial and agricultural production, geophysics, and petroleum exploration fields. The CT scanning system has a fast imaging speed and a high accuracy, thus may fully present three-dimensional information of an inspected site.

With the continuous expansion of CT application scenarios, there is an increasing demand for fast CT in more and more fields, such as CT scans of active tissues and organs in clinical diagnosis. By using slip ring technologies, the spiral CT greatly reduces scanning duration, but the rotational speed of the slip ring has almost reached its limit, making it difficult to further shorten the scanning duration. The emergence of multi-source spiral CT has further shortened the CT scanning duration, and the multi-source spiral CT has obvious advantages in scanning speed. The more sources there are, the less the scanning duration. However, the multi-source system also brings challenges such as high costs and large amounts of data.

In recent years, the emergence of distributed light sources has provided the possibility to further shorten the scanning duration. Distributed light sources avoid the use of slip rings and only require sequential exposure for imaging, completely avoiding mechanical motions. However, existing distributed light source scanning schemes commonly suffer from limited angle scanning, data truncation, and motion artifacts, which increase the difficulty of data processing and image reconstruction, while the imaging quality may also be affected.

It should be noted that the above information disclosed in this section is only for the purpose of understanding the background of the inventive concept of the present disclosure, and therefore, the above information may include information that does not constitute the related art.

SUMMARY

The present disclosure provides a CT imaging system, including: an imaging channel, at least a part of the imaging channel extending along a first direction, wherein the imaging channel is configured to place an object to be imaged in an imaging process; M distributed X-ray sources arranged along a circumferential direction of the imaging channel, wherein at least one of the M distributed X-ray sources includes q target spots for emitting rays, where M and q are positive integers greater than or equal to 2; and N detectors arranged along the circumferential direction of the imaging channel, wherein the N detectors are configured to detect rays emitted from the M distributed X-ray sources and penetrating the object to be imaged, where N is a positive integer greater than M, wherein the N detectors include first sub-detectors and second sub-detectors; wherein the CT imaging system includes a plurality of imaging components, each of the plurality of imaging components includes at least one distributed X-ray source and at least one first sub-detector; in the same imaging component, the distributed X-ray source and the first sub-detector are arranged face-to-face in a radial direction of the imaging channel, a plurality of target spots of the distributed X-ray source and at least a part of the first sub-detector are located in a same plane perpendicular to the first direction, and the radial direction is perpendicular to the first direction; and wherein at least one second sub-detector is provided on at least one side of the at least one distributed X-ray source in the first direction.

According to some exemplary embodiments, the distributed X-ray source includes a first side and a second side opposite to each other in the first direction, and the first side is located downstream of the second side; and wherein in the first direction, each of M' distributed X-ray sources is provided with at least one second sub-detector on the first side; or in the first direction, each of the M' distributed X-ray sources is provided with at least one second sub-detector on the second side; or in the first direction, each of M' distributed X-ray sources is provided with at least one second sub-detector on the first side and at least one second sub-detector on the second side, where M' is greater than or equal to 1 and less than or equal to M.

According to some exemplary embodiments, the CT imaging system includes a plurality of source-detector components, the source-detector component includes the distributed X-ray source and at least one second sub-detector; in the same source-detector component, an orthographic projection of the distributed X-ray source in the first direction overlaps at least partially with an orthographic projection of the at least one second sub-detector in the first direction.

According to some exemplary embodiments, in the same distributed X-ray source, the q target spots are arranged at intervals along a first straight line.

According to some exemplary embodiments, in the same source-detector component, a dimension of the distributed X-ray source along an extension direction of the first straight line is equal to a dimension of the second sub-detector along the extension direction of the first straight line.

According to some exemplary embodiments, the M distributed X-ray sources are uniformly arranged at equal intervals along the circumferential direction of the imaging channel; and/or the CT imaging system includes M first sub-detectors uniformly arranged at equal intervals along the circumferential direction of the imaging channel.

According to some exemplary embodiments, a ray angular coverage range of the M distributed X-ray sources is between 90° and 120°; or a ray angular coverage range of the M distributed X-ray sources is between 120° and 150°; or a ray angular coverage range of the M distributed X-ray sources is between 140° and 160°.

According to some exemplary embodiments, the CT imaging system includes M first sub-detectors, and the M distributed X-ray sources and the M first sub-detectors are alternately arranged along the circumferential direction of the imaging channel.

According to some exemplary embodiments, the CT imaging system includes M first sub-detectors; and along the circumferential direction of the imaging channel, the M distributed X-ray sources and the M first sub-detectors respectively enclose to form closed or approximately closed polygons.

According to some exemplary embodiments, the CT imaging system includes 2M second sub-detectors.

According to some exemplary embodiments, in the same imaging component, the q target spots of the distributed X-ray source are arranged at intervals along a first straight line, the distributed X-ray source has a first dimension along an extension direction of the first straight line, the first sub-detector has a second dimension in a direction parallel to the extension direction of the first straight line, and the first dimension is smaller than the second dimension.

According to some exemplary embodiments, the second dimension of the first sub-detector is greater than or equal to a dimension of an area of interest of the object to be imaged along the direction parallel to the extension direction of the first straight line.

According to some exemplary embodiments, the second dimension of the first sub-detector satisfies the following equation: $LD=d*p$, where $LD$ is the second dimension of the first sub-detector, $d$ is a movement distance of the object to be imaged in the first direction within an exposure period, and $p$ is a ray amplification ratio.

According to some exemplary embodiments, the first sub-detector includes a plurality of rows of detector units densely arranged along the first direction; or the first sub-detector includes a plurality of rows of detector units sparsely arranged along the first direction; or the first sub-detector includes a plurality of rows of detector units, at least two of the plurality of rows of detector units are densely arranged in the first direction in a middle area of the first sub-detector; and at least two other of the plurality of rows of detector units are sparsely arranged in the first direction on two side areas of the first sub-detector.

According to some exemplary embodiments, the second sub-detector includes a plurality of rows of detector units densely arranged along the first direction; or the second sub-detector includes a plurality of rows of detector units sparsely arranged along the first direction; or the second sub-detector includes a plurality of rows of detector units, at least two of the plurality of rows of detector units are densely arranged in the first direction in a middle area of the second sub-detector, and at least two other of the plurality of rows of detector units are sparsely arranged in the first direction on two side areas of the second sub-detector.

According to some exemplary embodiments, the plurality of rows of detector units include a middle row of detector units located at a middle position of the plurality of rows of detector units in the first direction; and wherein in the same imaging component, a plurality of target spots of the distributed X-ray source and the middle row of detector units of the first sub-detector are located in a same plane perpendicular to the first direction.

According to some exemplary embodiments, in a plane perpendicular to the first direction, the M distributed X-ray sources and the N detectors enclose to form a closed or approximately closed polygon.

According to some exemplary embodiments, a scanning imaging area of the CT imaging system is located in a middle area of the polygon.

According to some exemplary embodiments, the CT imaging system further includes a conveying mechanism located in the imaging channel, wherein the conveying mechanism is located in the middle area of the polygon.

According to embodiments of the present disclosure, by providing the second sub-detector to collaborate with the first sub-detector, it is possible to receive rays from the distributed X-ray sources from different angles or positions, thereby expanding a detection range. Such three-dimensional spatial arrangement breaks through the existing design of circumferential arrangement, enabling the imaging system to detect from multiple angles simultaneously, improving the accuracy and breadth of detection, and effectively solving the problems of limited angle scanning and data truncation.

BRIEF DESCRIPTION OF THE DRAWINGS

The above and other objectives, features, and advantages of the present disclosure will become clearer through the following description of embodiments of the present disclosure with reference to the accompanying drawings, in which:

FIG. 1 is a schematic structural diagram of a ray scanning imaging system according to some exemplary embodiments of the present disclosure;
FIG. 2 is a schematic structural diagram of a CT imaging system based on a three-stage coplanar scanning structure according to some exemplary embodiments of the present disclosure;

FIG. 3A is a schematic three-dimensional diagram of the CT imaging system based on FIG. 2 according to some exemplary embodiments of the present disclosure, which illustrates an arrangement of a plurality of distributed X-ray sources and a plurality of detectors;

FIG. 3B is a schematic diagram of an imaging light path of the CT imaging system based on FIG. 2 according to some exemplary embodiments of the present disclosure;

FIG. 4A is a schematic plan view illustrating an arrangement of distributed X-ray sources and detectors included in a CT imaging system according to some exemplary embodiments of the present disclosure, where two distributed X-ray sources and two detectors are shown;

FIG. 4B is a schematic plan view illustrating an arrangement of distributed X-ray sources and detectors included in a CT imaging system according to some exemplary embodiments of the present disclosure, where five distributed X-ray sources and five detectors are shown;

FIG. 4C is a schematic plan view illustrating an arrangement of distributed X-ray sources and detectors included in a CT imaging system according to some exemplary embodiments of the present disclosure, where a plurality of distributed X-ray sources and a plurality of detectors are shown;

FIG. 5A is a schematic plane view illustrating an arrangement of distributed X-ray sources and detectors included in a CT imaging system according to some exemplary embodiments of the present disclosure, where the distributed X-ray sources and detectors are respectively arranged in circumferential directions, and a ring where the distributed X-ray sources are located is an outer ring;

FIG. 5B is a schematic plane view illustrating an arrangement of distributed X-ray sources and detectors included in a CT imaging system according to some exemplary embodiments of the present disclosure, where the distributed X-ray sources and detectors are respectively arranged in circumferential directions, and a ring where the distributed X-ray sources are located is an inner ring;

FIG. 6 shows a schematic three-dimensional diagram of a CT imaging system according to some exemplary embodiments of the present disclosure, where detectors include two types of detectors;

FIG. 7 is a schematic diagram of an imaging light path of the CT imaging system based on FIG. 6 according to some exemplary embodiments of the present disclosure;

FIG. 8 is a schematic diagram of an imaging light path of a CT imaging system according to some other exemplary embodiments of the present disclosure;

FIG. 9A is a schematic plan view illustrating an arrangement of distributed X-ray sources and detectors included in a CT imaging system according to some other exemplary embodiments of the present disclosure, where two distributed X-ray sources and two detectors are shown;

FIG. 9B is a schematic plan view illustrating an arrangement of distributed X-ray sources and detectors included in a CT imaging system according to some other exemplary embodiments of the present disclosure, where five distributed X-ray sources and five detectors are shown;

FIG. 9C is a schematic plan view illustrating an arrangement of distributed X-ray sources and detectors included in a CT imaging system according to some other exemplary embodiments of the present disclosure, where a plurality of distributed X-ray sources and a plurality of detectors are shown;

FIG. 9D is a schematic plan view illustrating an arrangement of distributed X-ray sources and detectors included in a CT imaging system according to some other exemplary embodiments of the present disclosure, where the distributed X-ray sources and the detectors are respectively arranged in circumferential directions, and a ring where the distributed X-ray sources are located is an outer ring;

FIG. 9E shows a schematic diagram of an arrangement of a distributed X-ray source and a second sub-detector according to some embodiments of the present disclosure;

FIG. 9F shows a schematic diagram of an arrangement of a distributed X-ray source and a second sub-detector according to some other embodiments of the present disclosure;

FIG. 10A shows a schematic diagram of a layout design of dense detector arrangement according to some embodiments of the present disclosure;

FIG. 10B shows a schematic diagram of a layout design of sparse detector arrangement according to some other embodiments of the present disclosure;

FIG. 10C shows a schematic diagram of a layout design of hybrid detector arrangement according to some other embodiments of the present disclosure;

FIG. 11A shows a schematic diagram of a ray angular coverage range in a CT imaging system according to some embodiments of the present disclosure;

FIG. 11B shows a schematic diagram of a ray angular coverage range in a CT imaging system according to some other embodiments of the present disclosure;

FIG. 12A shows a schematic diagram of a valid imaging area in a CT imaging system according to some embodiments of the present disclosure;

FIG. 12B shows a schematic diagram of a valid imaging area in a CT imaging system according to some other embodiments of the present disclosure;

FIG. 13 is a schematic three-dimensional diagram of a CT imaging system according to some exemplary embodiments of the present disclosure, where target spots of the distributed X-ray source are obliquely arranged;

FIG. 14A is a schematic diagram of a distributed X-ray source of a CT imaging system according to some exemplary embodiments of the present disclosure;

FIG. 14B is a schematic diagram of a distributed X-ray source of a CT imaging system according to some other exemplary embodiments of the present disclosure;

FIG. 14C, FIG. 14D and FIG. 14E respectively show schematic diagrams of an arrangement of a distributed X-ray source and a second sub-detector according to some embodiments of the present disclosure, where FIG. 14C shows that second sub-detectors are provided on two sides of a distributed X-ray source, FIG. 14D shows that a second sub-detector is provided on an upper side of a distributed X-ray source, and FIG. 14E shows that a second sub-detector is provided on a lower side of a distributed X-ray source;

FIG. 15A shows a schematic diagram illustrating an arrangement of a second sub-detector according to some embodiments of the present disclosure;

FIG. 15B shows a schematic diagram of an arrangement of a second sub-detector according to some other embodiments of the present disclosure;

FIG. 16 shows a flowchart of an imaging method for a CT imaging system according to some exemplary embodiments of the present disclosure;

FIG. 17 shows a schematic diagram of a projection plane of simultaneous beam emitting of a plurality of target spots in a CT imaging system according to some exemplary embodiments of the present disclosure;

FIG. 18 shows a schematic diagram of a projection surface of time-division beam emitting of a plurality of target spots in a CT imaging system according to some exemplary embodiments of the present disclosure;

FIG. 19A is an image obtained using an imaging system and an imaging method in the related art;

FIG. 19B is an image obtained using a CT imaging system and an imaging method provided in embodiments of the present disclosure; and

FIG. 20 schematically shows a block diagram of an electronic apparatus suitable for implementing the imaging method according to embodiments of the present disclosure.

## DETAILED DESCRIPTION OF EMBODIMENTS

Hereinafter, embodiments of the present disclosure will be described with reference to the accompanying drawings. However, it should be understood that, these descriptions are merely exemplary and not intended to limit the scope of the present disclosure. In the following detailed descriptions, for ease of description, many specific details are elaborated to provide a comprehensive understanding of embodiments of the present disclosure. However, it is evident that one or more embodiments may also be implemented without these specific details. Furthermore, in the following descriptions, descriptions of well-known structures and techniques are omitted to avoid unnecessary confusion of the concepts of the present disclosure.

The terms used here are only for describing specific embodiments and are not intended to limit the present disclosure. The terms "including", "comprising"', etc. used herein indicate a presence of described features, steps, operations, and/or components, but do not exclude a presence or addition of one or more other features, steps, operations, or components.

All terms used herein (including technical and scientific terms) have meanings commonly understood by those skilled in the art, unless otherwise defined. It should be noted that the terms used here should be interpreted as having meanings consistent with the context of the specification, and should not be interpreted in an idealized or overly rigid manner.

When expressions such as "at least one of A, B, or C" are used, they may generally be interpreted according to the meaning commonly understood by those skilled in the art (for example, "a system having at least one of A, B, or C" may include but not be limited to a system having A alone, a system having B alone, a system having C alone, a system having A and B, a system having A and C, a system having B and C, and/or a system having A, B, and C).

It should be noted that herein, computed tomography (CT) imaging refers to that, after a tomography scan is performed on an object to be imaged using rays, an analog signal is received by a detector and converted into a digital signal, an attenuation coefficient of each pixel is calculated by an electronic computer, and an image is reconstructed so as to present a tomographic structure of various sites of the object to be imaged.

Hereinafter, embodiments of the present disclosure will be described in detail by taking a CT scanning imaging system for luggage as an example. It should be understood that, embodiments of the present disclosure are not limited to the CT scanning imaging scene of luggage, and may be applied to various scanning imaging scenarios. For example, embodiments of the present disclosure may be applied to scanning imaging scenarios that include various different inspection objects, including but not be limited to: vehicle scanning imaging, luggage/package scanning imaging, human or animal scanning imaging, organ/tissue scanning imaging, small object scanning imaging, large object scanning imaging such as containers, etc. It should be noted that, the descriptions of the scanning imaging scenarios here are not exhaustive, and the following exemplary descriptions should not be understood as limiting the scope of protection of the present disclosure.

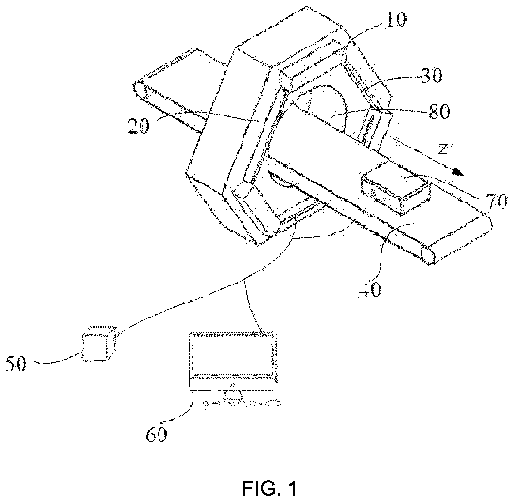

FIG. 1 is a schematic structural diagram of a ray scanning imaging system according to some exemplary embodiments of the present disclosure. In FIG. 1, a CT imaging system based on X-ray scanning is illustrated as an example of the ray scanning imaging system. As shown in FIG. 1, the CT imaging system according to embodiments includes: a gantry 20, a conveying mechanism 40, a controller 50, a data processing device 60 (such as a computer), an imaging channel 80, etc. The gantry 20 includes a X-ray source 10 such as an X-ray machine that emits X-rays for inspection, and a detection and acquisition device 30. The imaging channel 80 extends along a first direction z and is used to place an object 70 to be imaged in an imaging process. For example, at least a part of a carrying device may be provided in the imaging channel 80, and the object 70 to be imaged may be placed on the carrying device. In a scanning process, the object 70 to be imaged may be placed still in an imaging area. For another example, at least a part of the conveying mechanism 40 is placed in the imaging channel 80. The conveying mechanism 40 carries the object 70 to be imaged (such as inspected luggage) to pass through a scanning area between the X-ray source 10 and the detection and acquisition device 30 of the gantry 20. The conveying mechanism 40 may drive the object 70 to be imaged to move in the first direction z, while rays emitted by the X-ray source 10 may penetrate the object 70 to be imaged so as to perform CT scanning on the object 70 to be imaged. The detection and acquisition device 30 includes, for example, a detector (such as a flat panel detector) and a data collector that adopt structure of module, for detecting the rays transmitted through the object 70 to be imaged, obtaining analog signals, and converting the analog signals into digital signals, thereby outputting projection data for X-rays of the object 70 to be imaged. The controller 50 is used to control a synchronous operation of various parts of the entire system. The data processing device 60 is used to process the data collected by the data collector, process and reconstruct the data, and output a result.

As shown in FIG. 1, the X-ray source 10 is placed on one side of the object 70 to be imaged; the detection and acquisition device 30, including a detector and a data collector, is placed on the other side of the object 70 to be imaged, for obtaining transmission data and/or multi-angle projection data of the object 70 to be imaged. The data collector includes a data amplifying shaping circuit, which may operate in either (current) integration mode or pulse (counting) mode. A data output cable of the detection and acquisition device 30 is connected to the controller 50 and the data processing device 60, and the acquired data is stored in the data processing device 60 according to a trigger command.

In embodiments of the present disclosure, a ray scanning imaging module may be deployed in the data processing device 60, the ray scanning imaging module is provided with an image recognition model or a target recognition model. The image recognition model or the target recognition model may be used in the ray scanning imaging module to recognize the acquired on-site data (such as ray scanning images), and the recognition may include target detection, such as detecting target objects or objects of interest in the ray scanning images. For example, the target object or object of interest may be various prohibited items. It should be understood that, a specific category of the target object or object of interest depends on a scanning imaging site arranged by the ray scanning imaging system, or in other words, the specific category of the target object or object of interest is determined by a user of the ray scanning imaging system according to specific scanning imaging desires, which may be dynamically adjusted according to the scanning imaging desires.

In embodiments of the present disclosure, the X-ray source 10 may be, for example, an X-ray machine. An appropriate X-ray machine target spot size may be determined according to an imaging resolution. In other embodiments, an X-ray machine may not be used, but a linear accelerator or the like may be used to generate an X-ray beam.

The detection and acquisition device 30 includes an X-ray detector and a data acquisition circuit, etc. A solid-state detector, a gas detector, or other detectors may be used as the X-ray detector, which is not limited in embodiments of the present disclosure. The data acquisition circuit includes a readout circuit, an acquisition trigger circuit, a data transmitting circuit, etc.

A combination of the controller 50 and the data processing device 60 includes, for example, a computer installed with a control program and a data processing program, responsible for controlling an operation process of the CT imaging device, including mechanical rotation, electrical control, safety interlock control, etc., reconstructing CT images based on the projection data, training the image recognition model or the target recognition model, and recognizing the ray scanning images using the trained image recognition model or target recognition model, etc.

Furthermore, embodiments of the present disclosure will be described in detail by taking the CT imaging system with a three-stage scanning structure as an example. It should be understood that, the following CT imaging system with a three-stage coplanar scanning structure is an exemplary CT imaging system for ease of description. It should be understood to those skilled in the art that, numbers and positions of the X-ray sources and the detectors may be adjusted according to specific desires to adapt to objects to be imaged of different sizes and shapes.

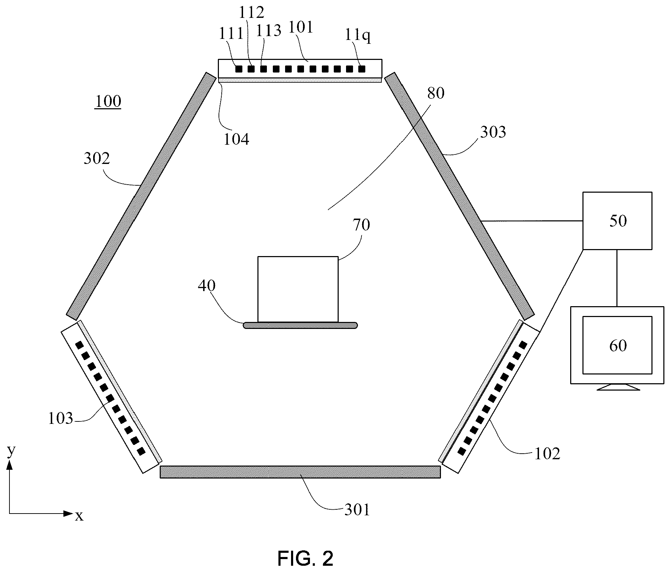

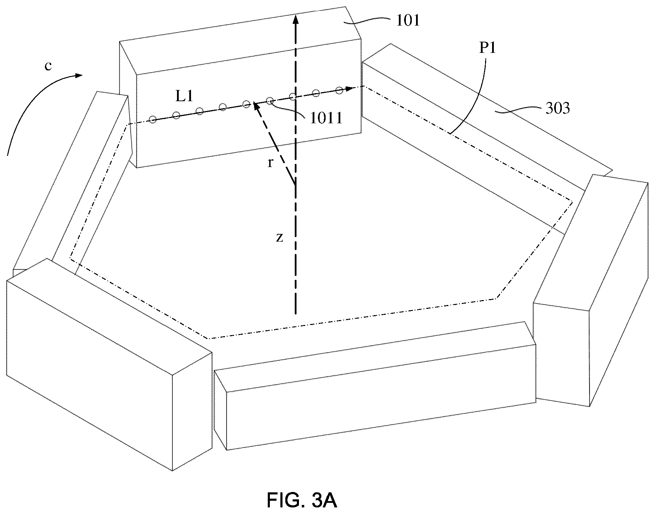

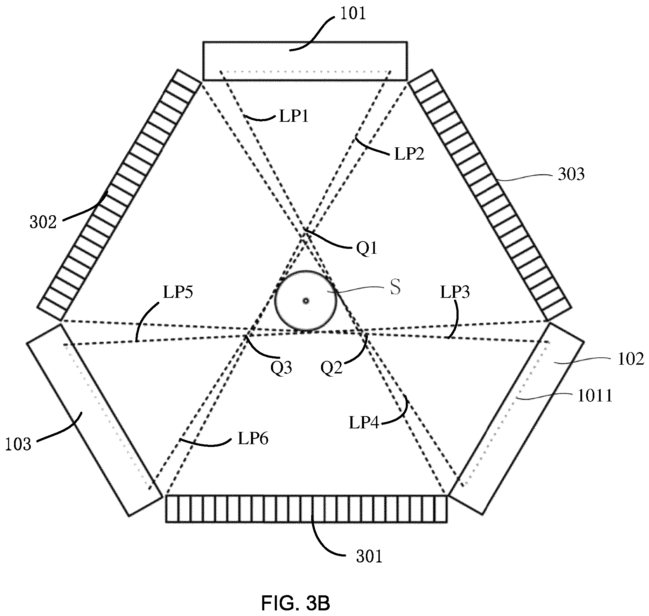

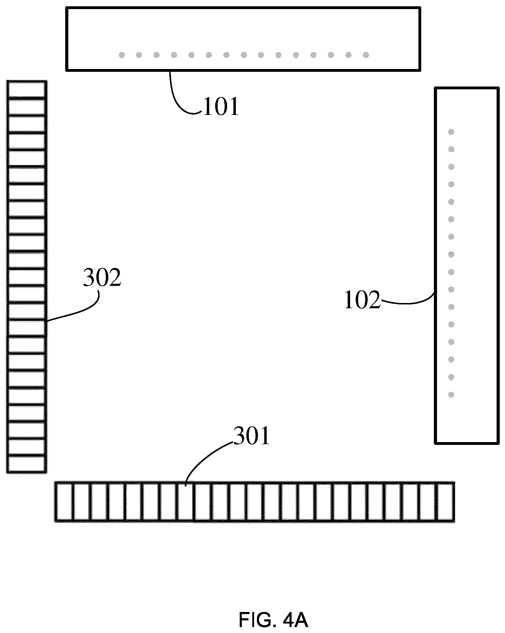

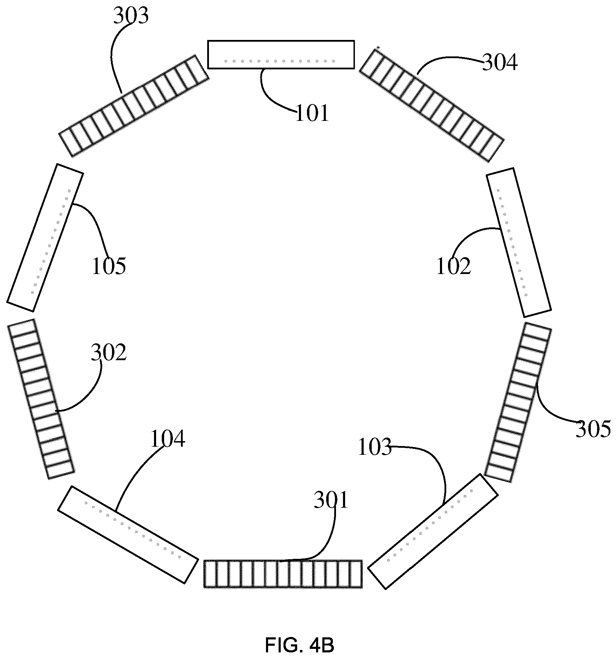

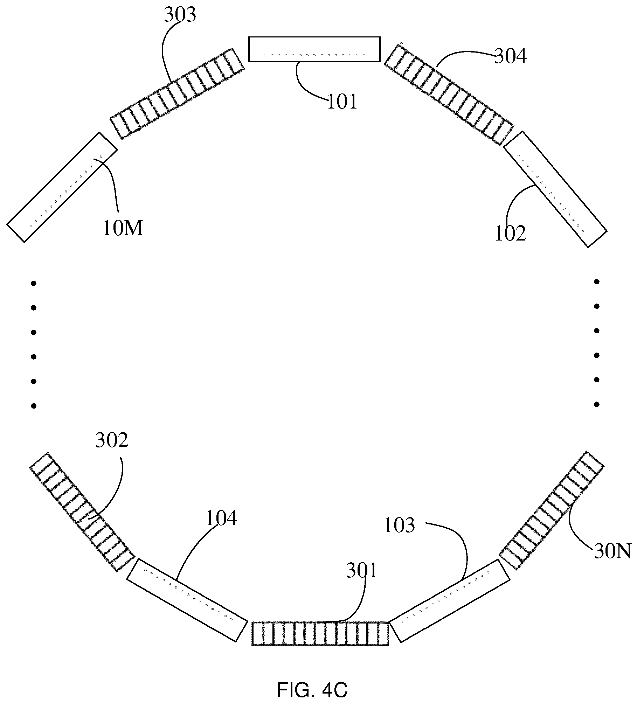

FIG. 2 is a schematic structural diagram of a CT imaging system based on a three-stage coplanar scanning structure according to some exemplary embodiments of the present disclosure. FIG. 3A is a schematic three-dimensional diagram of the CT imaging system based on FIG. 2 according to some exemplary embodiments of the present disclosure, which illustrates an arrangement of a plurality of distributed X-ray sources and a plurality of detectors. FIG. 3B is a schematic diagram of an imaging light path of the CT imaging system based on FIG. 2 according to some exemplary embodiments of the present disclosure. FIG. 4A is a schematic plan view illustrating an arrangement of distributed X-ray sources and detectors included in a CT imaging system according to some exemplary embodiments of the present disclosure, where two distributed X-ray sources and two detectors are shown; FIG. 4B is a schematic plan view illustrating an arrangement of distributed X-ray sources and detectors included in a CT imaging system according to some exemplary embodiments of the present disclosure, where five distributed X-ray sources and five detectors are shown; FIG. 4C is a schematic plan view illustrating an arrangement of distributed X-ray sources and detectors included in a CT imaging system according to some exemplary embodiments of the present disclosure, where a plurality of distributed X-ray sources and a plurality of detectors are shown.

Referring to FIG. 1, FIG. 2, FIG. 3A, FIG. 3B, FIG. 4A, FIG. 4B and FIG. 4C, in some exemplary embodiments, the X-ray source 10 of the CT imaging system 100 may include a plurality of distributed X-ray sources, such as M distributed X-ray sources, where M is a positive integer greater than or equal to 2. In the exemplary embodiments shown in FIG. 2, FIG. 3A and FIG. 3B, M=3, that is, three distributed X-ray sources are provided. In the exemplary embodiment shown in FIG. 4A, M=2, that is, two distributed X-ray sources are provided. In the exemplary embodiment shown in FIG. 4B, M=5, that is, five distributed X-ray sources are provided. In the exemplary embodiment shown in FIG. 4C, M is multiple, that is, a plurality of distributed X-ray sources are provided. In the present disclosure, for ease of description, M distributed X-ray sources are numbered as a first distributed X-ray source 101, a second distributed X-ray source 102, a third distributed X-ray source 103, ..., a M-th distributed X-ray source 10M.

In embodiments of the present disclosure, the distributed X-ray source includes a plurality of target spots (also known as focal points). For example, a plurality of X-ray target spots may be densely arranged within a vacuum chamber of a ray tube. Compared with an existing single X-ray source, the distributed X-ray source has a plurality of separate ray emission points, which may operate separately and be controlled separately. For example, the distributed X-X-ray source may integrate hundreds of ray target spots within a single tube, each of the ray target spots may be separately controlled and quickly switched as needed. For example, an emission of each target spot may be precisely controlled, including parameters such as ray intensity and emission time, so that in CT scanning, a ray output may be flexibly adjusted according to different scanning sites, object densities, and other factors, thereby obtaining higher quality images. For example, the intensity distribution of X-ray beams may be modulated by controlling ray intensities of different target spots to meet imaging needs of different objects. For another example, by using a distributed X-ray source with a plurality of target spots, fast scanning imaging capabilities may be achieved. As a plurality of ray target spots are provided, the distributed X-ray source may simultaneously or rapidly scan target objects from a plurality of different positions, greatly improving a scanning speed. Compared to an existing CT scanning method, complete image information of objects may be obtained in a shorter duration, which is of great significance for scenes such as medical emergencies, security checks, etc., that require fast imaging. For yet another example, high-quality imaging effects, collaborative operate of target spots, and precise control capabilities may be achieved, enabling the distributed X-ray source to provide images with higher resolution and lower noise. When imaging complex objects or micro structures, internal structures and details of the objects may be presented more clearly, providing more accurate information for doctors' diagnosis or other application scenarios.

Referring to FIG. 1, FIG. 2, FIG. 3A, FIG. 3B, FIG. 4A, FIG. 4B and FIG. 4C, in some exemplary embodiments, the detection and acquisition device 30 of the CT imaging system 100 may include a plurality of detectors, such as N detectors, where N is a positive integer greater than or equal to 2. For example, N is a positive integer greater than or equal to M. In the exemplary embodiments shown in FIG. 2, FIG. 3A and FIG. 3B, N=3, that is, three detectors are provided. In the exemplary embodiment shown in FIG. 4A, N=2, that is, two detectors are provided. In the exemplary embodiment shown in FIG. 4B, N=5, that is, 5 detectors are provided. In the exemplary embodiment shown in FIG. 4C, N is multiple, that is, a plurality of detectors are provided. In the present disclosure, for ease of description, N detectors are numbered as a first detector 301, a second detector 302, a third detector 303, ..., a N-th detector 30N.

Referring to FIG. 2, FIG. 3A, FIG. 3B, FIG. 4A, FIG. 4B and FIG. 4C, in some exemplary embodiments, at least one distributed X-ray source among the M distributed X-ray sources includes q target spots 1011, where q is a positive integer greater than or equal to 2. For example, the M distributed X-ray sources each include two or more target spots. In the embodiment shown in FIG. 2, q=11, that is, the distributed X-ray source includes 11 target spots. In the present disclosure, for ease of description, the q target spots 1011 are numbered as a first target spot 111, a second target spot 112, a third target spot 113, ..., a q-th target spot 11q. For example, the q target spots are activated in a predetermined order to emit ray.

It should be understood that, the 2, 3, or 5 distributed X-ray sources and the 2, 3, or 5 detectors described herein are exemplary embodiments and should not be construed as limitations on the numbers of the distributed X-ray sources and the detectors in embodiments of the present disclosure. In other embodiments, fewer distributed X-ray sources and detectors with the number of 4, or more distributed X-ray sources and detectors with the number of 6, 8, 10, or more may be included in the CT imaging system. When two or more distributed X-ray sources and two or more detectors are provided, it is possible to simultaneously or sequentially emit and receive rays from different angles and positions, thereby improving imaging speed and resolution. Alternatively, three, four, or five distributed X-ray sources and three, four, or five detectors are provided, and a combination of three or more distributed X-ray sources and detectors may provide more comprehensive and detailed scanning for the imaging of complex objects. For another example, six distributed X-ray sources and six detectors are provided, and a ray coverage over a larger range may be achieved, which has significant advantages for imaging large objects or specific application scenarios that require high resolution. In some cases, more than eight distributed X-ray sources and eight detectors may be provided. The configuration of more than eight distributed X-ray sources and more than eight detectors may achieve more complex scanning modes and multi-angle imaging, providing richer image information for security check scenarios, doctor diagnosis, and researcher analysis. It should be understood that, with an increase in the number of distributed X-ray sources, the CT imaging system may achieve more precise imaging, making it easier to observe micro structures and perform high-precision detection tasks. In embodiments of the present disclosure, the number of distributed X-ray sources and the number of detectors may be flexibly adjusted according to specific application requirements to meet CT imaging requirements in different fields and scenarios.

It should also be understood that, the distributed X-ray source shown in FIG. 2 includes 11 target spots, and the numbers of target spots shown in other drawings are exemplary embodiments and should not be understood as limitations on the number of target spots in embodiments of the present disclosure. In other embodiments, the distributed X-ray source of the CT imaging system may include fewer target spots with the number of less than 10, or more target spots with the number of 20, 50, 100, hundreds or more. In embodiments of the present disclosure, the number of target spots of the distributed X-ray source may be flexibly adjusted according to specific application requirements to meet the CT imaging requirements in different fields and scenarios.

It should be noted that unless otherwise specified, the expressions "first", "second", "third", etc., described herein are intended to refer to different components such as distributed X-ray sources, target spots, and detectors more conveniently and quickly in description processes, and should not be understood as any form of limitation on structures of distributed X-ray sources, target spots, and detectors. In embodiments of the present disclosure, the structures of the first distributed X-ray source 101, the second distributed X-ray source 102, the third distributed X-ray source 103, ..., and the M-th distributed X-ray source 10M may be the same, or at least two of the distributed X-ray sources may have the same structure while at least two other of the distributed X-ray sources may have different structures. For example, the first distributed X-ray source 101, the second distributed X-ray source 102, the third distributed X-ray source 103, ..., the M-th distributed X-ray source 10M may include the same number of target spots; or at least one of distributed X-ray sources may include the number of target spots different from at least one other of distributed X-ray sources; or any two distributed X-ray sources may include different numbers of target spots. In embodiments of the present disclosure, the structures of the first detector 301, the second detector 302, the third detector 303, ..., the N-th detector 30N may be the same, or at least two of the detectors may have the same structure while at least two other of the detectors may have different structures.

It should also be noted that, the structure of the distributed X-ray source has high flexibility and diversity, and a design and a construction of the distributed X-ray source depend on various factors, including but not limited to specific application scenarios, imaging requirements, technical requirements, engineering feasibility, etc. Each distributed X-ray source may be separately optimized and adjusted according to actual conditions to achieve optimal performance. Whether in the fields of medical diagnosis, safety inspection, industrial testing, or scientific research, the structure of the distributed X-ray source may be customized according to different goals and tasks without being constrained by the simple numbering used here. Such numbering is for the convenience of distinguishing and discussing various distributed X-ray sources in text descriptions, and should not be misunderstood as providing any fixed patterns or limitations on the complex and exquisite structure.

Referring to FIG. 1, FIG. 2, FIG. 3A, FIG. 3B, FIG. 4A, FIG. 4B and FIG. 4C, herein, for the convenience of description, the first direction z, a second direction x, a third direction y, a circumferential direction c, and a radial direction r of the imaging channel are respectively described. For example, the first direction z corresponds to an extension direction of the imaging channel 80, or corresponds to a movement direction of the object 70 to be imaged when the object 70 to be imaged is moving. The second direction x intersects with the third direction y, and the first direction z is perpendicular to both the second direction x and the third direction y. For example, the second direction x corresponds to a width direction of the CT imaging system, and the third direction y corresponds to a height direction of the CT imaging system. The circumferential direction c of the imaging channel intersects with the radial direction r of the imaging channel, and the first direction z is perpendicular to both the circumferential direction c and radial direction r of the imaging channel. For example, the radial direction r of the imaging channel 80 corresponds to a direction in which a center of imaging channel 80 points towards an outer periphery of the imaging channel 80, and the circumferential direction c of the imaging channel 80 corresponds to a direction surrounding the imaging channel 80.

Referring to FIG. 2, FIG. 3A, FIG. 3B, FIG. 4A, FIG. 4B and FIG. 4C, the M distributed X-ray sources 101 to 10M are arranged along the circumferential direction c of imaging channel 80. For example, the M distributed X-ray sources 101 to 10M are arranged at intervals along the circumferential direction c of imaging channel 80.

Continuing to refer to FIG. 2, FIG. 3A, FIG. 3B, FIG. 4A, FIG. 4B and FIG. 4C, the N detectors 301 to 30N are arranged along the circumferential direction c of the imaging channel 80. For example, the N detectors 301 to 30N are arranged at intervals along the circumferential direction c of the imaging channel 80.

In some exemplary embodiments, the M distributed X-ray sources 101 to 10M and the N detectors 301 to 30N are alternately arranged along the circumferential direction c of the imaging channel 80. For example, the M distributed X-ray sources 101 to 10M are arranged at equal intervals along the circumferential direction c of the imaging channel 80, with one detector provided between two adjacent distributed X-ray sources. The N detectors 301 to 30N are arranged at equal intervals along the circumferential direction c of the imaging channel 80, with one distributed X-ray source provided between two adjacent detectors.

It should be understood that, FIG. 2, FIG. 3B, FIG. 4A, FIG. 4B and FIG. 4C respectively represent schematic plane diagrams perpendicular to the first direction z, that is, paper surfaces on which FIG. 2, FIG. 3B, FIG. 4A, FIG. 4B and FIG. 4C are located are perpendicular to the first direction z. As shown in FIG. 2, FIG. 3B, FIG. 4A, FIG. 4B and FIG. 4C, in the plane perpendicular to the first direction z, the M distributed X-ray sources 101 to 10M and the N detectors 301 to 30N surround to form a closed or approximately closed polygon. For example, in FIG. 2 and FIG. 3B, three distributed X-ray sources 101 to 103 and three detectors 301 to 303 surround to form a hexagon; in FIG. 4A, two distributed X-ray sources 101 and 102 and two detectors 301 and 302 surround to form a quadrilateral; in FIG. 4B, five distributed X-ray sources 101 to 105 and five detectors 301 to 305 surround to form a decagon; and in FIG. 4C, M distributed X-ray sources 101 to 10M and the N detectors 301 to 30N surround to form an (M+N) side polygon.

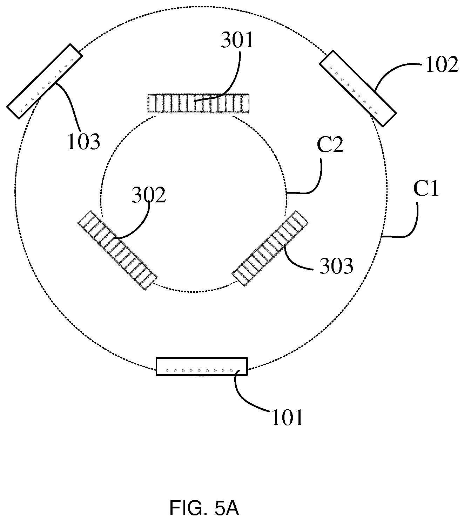

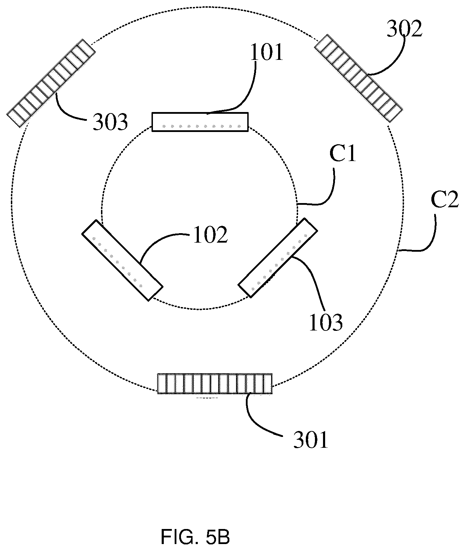

FIG. 5A is a schematic plane view illustrating an arrangement of distributed X-ray sources and detectors included in a CT imaging system according to some exemplary embodiments of the present disclosure, where the distributed X-ray sources and detectors are respectively arranged in a circumferential direction, and a ring where the distributed X-ray sources are located is an outer ring; FIG. 5B is a schematic plane view illustrating an arrangement of distributed X-ray sources and detectors included in a CT imaging system according to some exemplary embodiments of the present disclosure, where the distributed X-ray sources and detectors are respectively arranged in a circumferential direction, and a ring where the distributed X-ray sources are located is an inner ring.

Referring to FIG. 5A and FIG. 5B, the M distributed X-ray sources 101 to 10M are arranged along the circumferential direction c of the imaging channel 80. In an example, the M distributed X-ray sources 101 to 10M are arranged at intervals along the circumferential direction c of the imaging channel 80, and the M distributed X-ray sources 101 to 10M form a closed or approximate closed polygon.

Continuing to refer to FIG. 5A and FIG. 5B, the N detectors 301 to 30N are arranged along the circumferential direction c of the imaging channel 80. In an example, the N detectors 301 to 30N are arranged at intervals along the circumferential direction c of the imaging channel 80, and the N detectors 301 to 30N form a closed or approximate closed polygon.

In some exemplary embodiments, the M distributed X-ray sources 101 to 10M and the N detectors 301 to 30N are respectively arranged along the circumferential direction c of the imaging channel 80. For example, the M distributed X-ray sources 101 to 10M are arranged at equal intervals along the circumferential direction c of the imaging channel 80, approximately located on a first circumference C1. The N detectors 301 to 30N are arranged at equal intervals along the circumferential direction c of the imaging channel 80, approximately located on a second circumference C2. As shown in FIG. 5A, a radial size of the first circumference C1 may be greater than a radial size of the second circumference C2; and as shown in FIG. 5B, a radial size of the first circumference C1 may be smaller than a radial size of the second circumference C2.

It should be understood that, the M distributed X-ray sources 101 to 10M and the N detectors 301 to 30N respectively form a closed or approximately closed polygon. For ease of description, dashed lines shown in FIG. 5A and FIG. 5B are used to represent tangent circles of the closed or approximately closed polygons formed by the M distributed X-ray sources 101 to 10M and the N detectors 301 to 30N, that is, the first circumference C1 and the second circumference C2, so as to describe the size relationship and the position relationship between the geometric shapes formed by the M distributed X-ray sources 101 to 10M and the N detectors 301 to 30N.

It should be understood that, FIG. 5A and FIG. 5B are schematic plan views perpendicular to the first direction z, that is, a paper surface where FIG. 5A is located and a paper surface where FIG. 5B is located are perpendicular to the first direction z.

In some exemplary embodiments of the present disclosure, the CT imaging system 100 includes a plurality of imaging components.

It should be noted that herein, the term "imaging component" may refer to a component capable of imaging based on detected ray, which may include a X-ray source for emitting ray and a detector for detecting the ray emitted by the X-ray source, and performing imaging based on the ray detected by the detector.

For example, the imaging component may include at least one distributed X-ray source and at least one detector, which are arranged face-to-face in the radial direction r of the imaging channel 80 in the same imaging component. For example, in FIG. 2 and FIG. 3B, the first distributed X-ray source 101 and the first detector 301 are arranged face-to-face in the radial direction r of the imaging channel 80, forming an imaging component; the second distributed X-ray source 102 and the second detector 302 are arranged face-to-face in the radial direction r of the imaging channel 80, forming an imaging component; the third distributed X-ray source 103 and the third detector 303 are arranged face-to-face in the radial direction r of the imaging channel 80, forming an imaging component.

In some exemplary embodiments of the present disclosure, in the same imaging component, the target spots of the distributed X-ray source and at least a part of the detector are located in a same plane perpendicular to the first direction z.

In some exemplary embodiments of the present disclosure, q target spots of at least one distributed X-ray source are arranged at intervals along a straight line. By way of example, referring to FIG. 3A and FIG. 3B, the q target spots of the first distributed X-ray source 101 are arranged at intervals along the first straight line L1. The extension direction of the first straight line L1 may be perpendicular to both the first direction z and the radial direction r. In the same imaging component including the first distributed X-ray source 101 and the first detector 301, the first straight line L1 and at least a part of the first detector 101 (a part facing the first distributed X-ray source 101) are located in a same first plane P1 perpendicular to the first direction z.

In embodiments of the present disclosure, the CT imaging system adopts a source-detector coplanar structure. Specifically, the plurality of distributed X-ray sources and the plurality of detectors are located in the same plane perpendicular to the first direction z. By adopting the distributed X-ray source and the source detector coplanar design, more complete data may be obtained at different angles, thereby reducing signal loss, obtaining finer image details, and allowing for faster scanning.

It should be noted that herein, unless otherwise specified, expressions such as "source-detector coplanar", "coplanar", "located in the same plane" not only include that two components (such as the X-ray source and the detector) are located in a same geometric plane, but also include that two components (such as the X-ray source and the detector) are located in a same engineering plane. For example, the plane where the two components coexist may have a certain thickness, which may be determined by process dimensions of the components, for example, by a dimension of the target spot of the X-ray source along the first direction z. In a case where the detector includes a plurality of rows of detector units, the thickness may be determined by a dimension of a single row of detectors along the first direction z.

In some exemplary embodiments of the present disclosure, the target spots of the M distributed X-ray sources included in the CT imaging system are located in a same plane perpendicular to the first direction z. For example, referring to FIG. 3A and FIG. 3B, the q target spots of the first distributed X-ray source 101, the q target spots of the second distributed X-ray source 102, and the q target spots of the third distributed X-ray source 103 are located in the same first plane P1 perpendicular to the first direction z.

In embodiments of the present disclosure, an overall source-detector arrangement of the CT imaging system adopts a source-detector coplanar structure. By adopting the distributed X-ray source and the overall source-detector coplanar design, a compact structure and a size reduction of the CT imaging system are achieved, while allowing for more complete data to be obtained at different angles, thereby reducing signal loss, obtaining finer image details, and allowing for faster scanning.

For example, referring to FIG. 2, the CT imaging system 100 may further include a collimator 104. For example, the collimator 104 may be located in front of the distributed X-ray source to limit a direction and a range of ray beams emitted by the distributed X-ray source, ensuring that the ray beams can be accurately projected onto the object to be imaged, thereby reducing an interference of scattered rays and improving a clarity of imaging.

In embodiments of the present disclosure, the controller 50 may adjust the scanning mode of each target spot in the distributed X-ray source through several key parameters to achieve optimal image quality and imaging efficiency. For example, the controller 50 may control at least one of the following aspects of each target spot according to actual imaging requirements: an activation moment of each target spot, an emission range of each target spot, a beam emitting order of of each target spot, an emission intensity of each target spot, or a beam emitting combination of the target spots.

In embodiments of the present disclosure, the M distributed X-ray sources may be arranged at intervals along the circumferential direction of the imaging channel, distributed at different angles in the imaging area, for irradiating the object to be imaged from a plurality of directions to obtain perspective images at different angles; the N detectors may also be arranged at intervals along the circumferential direction of the imaging channel, and are arranged opposite to the M distributed X-ray sources, respectively, for receiving ray signals transmitted through the object to be imaged and converting the ray signals into electrical signals to obtain CT projection data.

For example, as shown in FIG. 3A, three distributed X-ray sources are distributed at different angles in a polygonal pattern in the imaging area. Such design may irradiate the object to be imaged with rays from a plurality of directions, thereby obtaining perspective images at different angles; three detectors are opposite to the distributed X-ray sources, respectively, and are arranged around the imaging area to receive the ray signals transmitted through the object to be imaged. A positional relationship between the detectors and the X-ray sources ensures that the system may obtain high-quality CT projection data.

As shown in FIG. 3B, dashed lines marked in the figure represent farthest paths of the imaging light paths of the X-rays emitted from the distributed X-ray sources to reach the detectors on the opposite side after passing through the object to be imaged. For example, for the first distributed X-ray source 101 located on an upper side, a light path LP1 represents a light path of rays emitted from the first target spot located on a leftmost side and incident onto a rightmost unit of the first detector 301; a light path LP2 represents a light path of rays emitted from the q-th target spot located on a rightmost side and incident onto a leftmost unit of the first detector 301, and the two light paths LP1 and LP2 intersect at a point Q1 in the plane P1 where the sources and the detectors are located. An area enclosed by the two light paths LP1 and LP2 and a detection surface of the first detector 301 is a first imaging area of the imaging component including the first distributed X-ray source 101 and the first detector 301. For the second distributed X-ray source 102 located on a left side, a light path LP3 represents a light path of rays emitted from the first target spot located on a topmost side and incident onto a lowermost unit of the second detector 302, and a light path LP4 represents a light path of rays emitted from the q-th target spot located on a lowermost side and incident onto a topmost unit of the second detector 302. The two light paths LP3 and LP4 intersect at a point Q2 in the plane P1 where the sources and the detectors are located. An area enclosed by the two light paths LP3 and LP4 and a detection surface of the second detector 302 is a second imaging area of the imaging component including the second distributed X-ray source 102 and the second detector 302. For the third distributed X-ray source 103 located on a right side, a light path LP5 represents a light path of rays emitted from the first target spot located on a topmost side and incident onto a lowermost unit of the third detector 303, and a light path LP6 represents a light path of rays emitted from the q-th target spot located on a lowermost side and incident onto a topmost unit of the third detector 303. The two light paths LP5 and LP6 intersect at a point Q3 in the plane P1 where the sources and detectors are located. An area enclosed by the two light paths LP5 and LP6 and a detection surface of the third detector 303 is a third imaging area of the imaging component including the third distributed X-ray source 103 and the third detector 303. An overlapping area of the first imaging area, the second imaging area, and the third imaging area forms a polygonal area, for example, in the example of FIG. 3B, an approximate triangular area is formed, and a maximum inscribed circle of the polygonal area forms a valid imaging area S.

Although the closed-loop structure in which the target spots of the distributed X-ray sources and the detectors are coplanar and distributed around the object to be imaged may bring convenience in design and operation, a limited coverage range of ray angles due to the sources and the detectors arranging at intervals and a limited length ratio of sources and detectors may lead to a problem of limited angle scanning. Thus, in the process of image reconstruction, relevant issues of limited angle reconstruction need to be considered, and an area of the object to be imaged is limited. In addition, due to the limited valid imaging area covered by the scanning paths, when a certain part of the object to be imaged exceed the valid imaging area S, the projection data of said part may not be obtained, resulting in missing or distorted information, that is, data truncation, in some areas of the reconstructed image. In practical applications, especially when detecting complex or high-density objects, the problem of data truncation may affect the comprehensiveness and accuracy of detection and imaging.

For another scanning imaging scheme for distributed light sources, the related art has proposed a scanning system in which the target spot of the X-ray source and the detector are distributed in different planes, that is, the target spot and the detector are distributed in two or more planes. In this scanning method, the object is scanned by the ray obliquely, resulting in the problem of data truncation.

On this basis, embodiments of the present disclosure further provide a CT imaging system, in which two types of detectors are provided, one type of detectors are as described above, which are provided face-to-face with the various distributed X-ray sources; and the other type of detectors are provide on a same side as the distributed X-ray sources. Herein, for the convenience of description, these two types of detectors are respectively referred to as a first sub-detector and a second sub-detector.

In some embodiments of the present disclosure, by providing a plurality of target spots of the distributed X-ray source and the first sub-detector in the same plane perpendicular to the imaging channel, and providing the second sub-detector on the same side as the X-ray source, the valid imaging area is expanded, the imaging quality of object edges and corners is improved, thereby achieving more complete and effective ray projection and reception, and improving the imaging efficiency.

For example, some embodiments of the present disclosure provide a CT imaging system, including: an imaging channel, where at least a part of the imaging channel extends along the first direction, and the imaging channel is used to place the object to be imaged in the imaging process; M distributed X-ray sources arranged at intervals along the circumferential direction of the imaging channel, where at least one distributed X-ray source includes q target spots configured to be activated in a predetermined order to emit rays, and where M and q are both positive integers greater than or equal to 2; and N detectors arranged at intervals along the circumferential direction of the imaging channel, where the N detectors are used to detect rays emitted from the M distributed X-ray sources and penetrating the object to be imaged, and where N is a positive integer greater than M. The detectors include a first sub-detector and a second sub-detector. The CT imaging system includes a plurality of imaging components, and the imaging component includes at least one distributed X-ray source and at least one first sub-detector. In the same imaging component, the distributed X-ray source and the first sub-detector are arranged face-to-face in the radial direction of the imaging channel. In the same imaging component, the plurality of target spots of the distributed X-ray source and at least a part of the first sub-detector are located in the same plane perpendicular to the first direction, and the radial direction is perpendicular to the first direction. At least one second sub-detector is provided on at least one side of at least one distributed X-ray source in the first direction.

In this embodiment, by providing the second sub-detector on the same side of the X-ray source, X-rays may be simultaneously received by sub-detectors including the first and second sub-detectors after penetrating the object to be imaged, thereby enhancing coverage of different angles, reducing data truncation or loss, and improving detection accuracy.

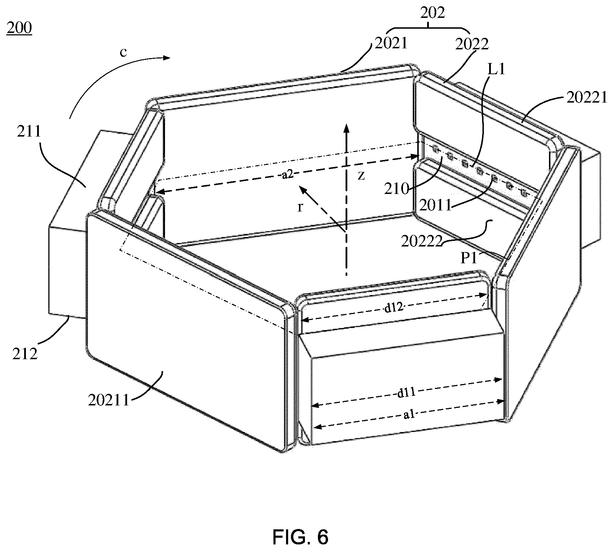

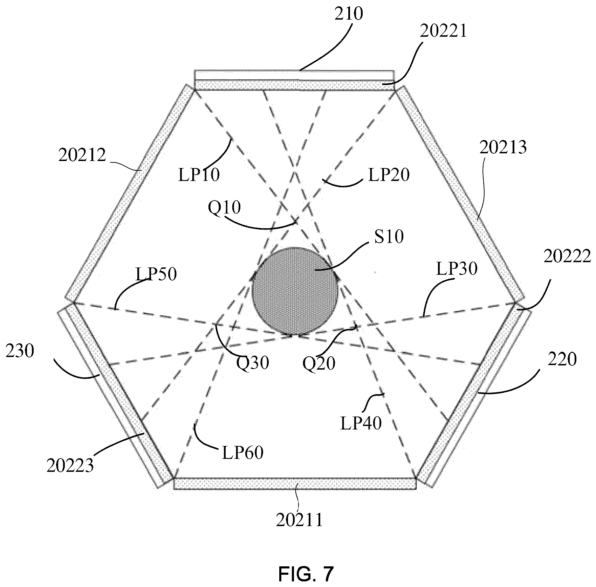

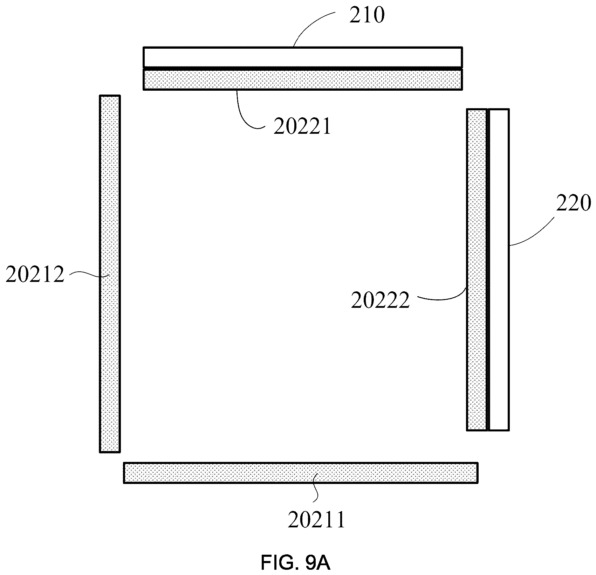

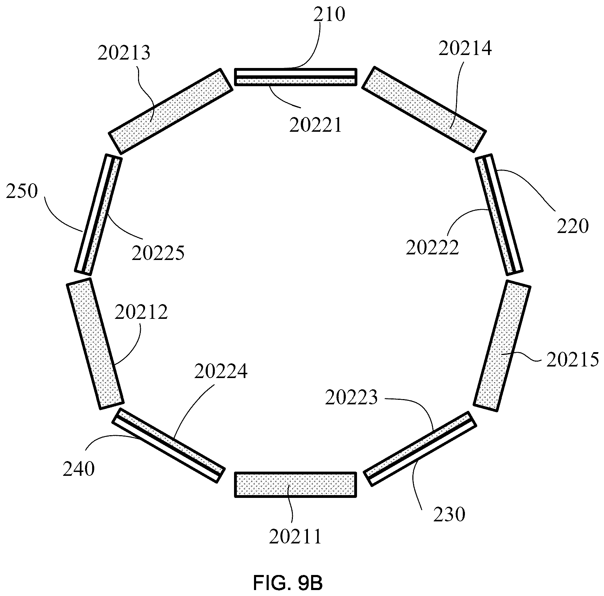

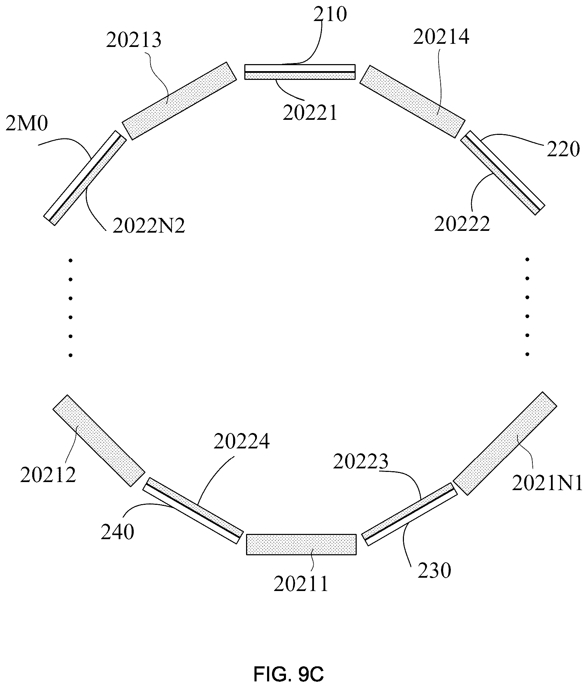

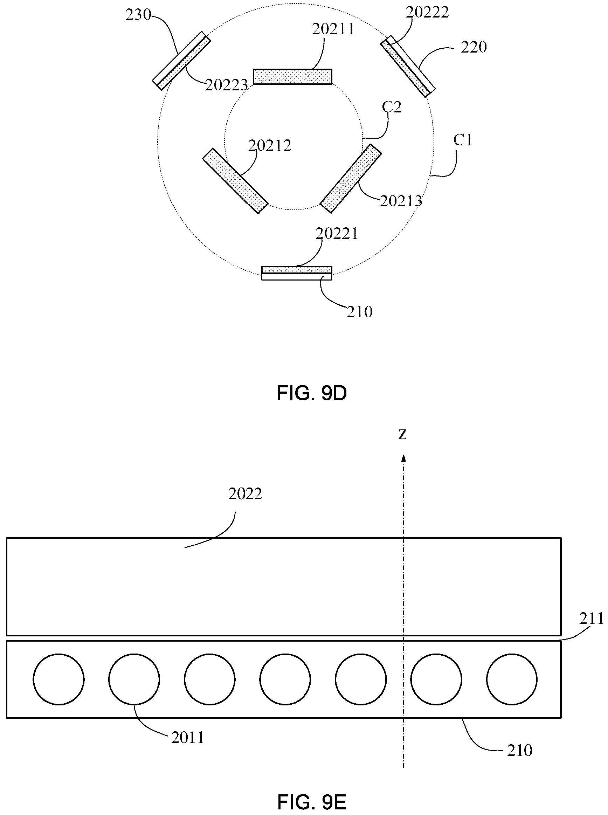

FIG. 6 shows a schematic three-dimensional diagram of a CT imaging system according to some exemplary embodiments of the present disclosure, where detectors include two types of detectors. FIG. 7 is a schematic diagram of an imaging light path of the CT imaging system based on FIG. 6 according to some exemplary embodiments of the present disclosure. FIG. 9A is a schematic plan view illustrating an arrangement of distributed X-ray sources and detectors included in a CT imaging system according to some other exemplary embodiments of the present disclosure, where two distributed X-ray sources and two detectors are shown; FIG. 9B is a schematic plan view illustrating an arrangement of distributed X-ray sources and detectors included in a CT imaging system according to some other exemplary embodiments of the present disclosure, where five distributed X-ray sources and five detectors are shown; FIG. 9C is a schematic plan view illustrating an arrangement of distributed X-ray sources and detectors included in a CT imaging system according to some other exemplary embodiments of the present disclosure, where a plurality of distributed X-ray sources and a plurality of detectors are shown; FIG. 9D is a schematic plan view illustrating an arrangement of distributed X-ray sources and detectors included in a CT imaging system according to some other exemplary embodiments of the present disclosure, where the distributed X-ray sources and the detectors are respectively arranged in circumferential directions, and a ring where the distributed X-ray sources are located is an outer ring.

It should be noted that, without conflict, the contents and features described in the previous text may be combined with the various embodiments described in the following, which will not be repeated in the various embodiments described in the following in order to save space. For example, in embodiments described below, a CT imaging system with a three-stage scanning structure is taken as an example to provide a detailed description of embodiments of the present disclosure. It should be understood that, the following three-stage scanning structure CT imaging system is an exemplary CT imaging system structure for ease of description. Those skilled in the art should understand that the numbers and positions of the X-ray sources and the detectors may be adjusted according to specific needs to adapt to the objects to be imaged of different sizes and shapes. For example, the cases of 3 distributed X-ray sources, 2 distributed X-ray sources, 5 distributed X-ray sources, and M distributed X-ray sources described above may be combined with the various embodiments described below.

Referring to FIG. 6, FIG. 7, FIG. 9A, FIG. 9B, FIG. 9C and FIG. 9D, in some exemplary embodiments, the X-ray source of the CT imaging system 200 may include a plurality of distributed X-ray sources such as M distributed X-ray sources, where M is a positive integer greater than or equal to 2. Herein, for the convenience of description, the M distributed X-ray sources are numbered as a first distributed X-ray source 210, a second distributed X-ray source 220, a third distributed X-ray source 230, ... and a M-th distributed X-ray source 2M0.

In some exemplary embodiments, the detection and acquisition device 30 of the CT imaging system 200 may include a plurality of detectors, such as N detectors 202, where N is a positive integer greater than or equal to 2. For example, N is a positive integer greater than or equal to M. In this embodiment, two types of detectors may be provided, that is to say, the detectors 202 may include a first sub-detector 2021 and a second sub-detector 2022. For example, the CT imaging system 200 may include N1 first sub-detectors 2021 and N2 second sub-detectors 2022. N is a sum of N1 and N2, where N1 is a positive integer greater than or equal to 2 and N2 is a positive integer greater than or equal to 2.

For example, N1 may be equal to M, that is, the number of the first sub-detectors is equal to the number of the distributed X-ray sources.

In the present disclosure, for the convenience of description, the N1 first sub-detectors are numbered as a 1st first sub-detector 20211, a 2nd first sub-detector 20212, a 3rd first sub-detector 20213, ... and N1-th first sub-detector 2021N1; the N2 second sub-detectors are numbered as a 1st second sub-detector 20221, a 2nd second sub-detector 20222, a 3rd second sub-detector 20223, ... and N2-th second sub-detector 2022N2.

In this embodiment, the CT imaging system 200 may include: an imaging channel 80, where at least a part of the imaging channel 80 extends along the first direction z, and the imaging channel 80 is used to place the object 70 to be imaged in the imaging process; M distributed X-ray sources arranged at intervals along the circumferential direction c of the imaging channel 80, where at least one distributed X-ray source includes q target spots 2011 configured to be activated in a predetermined order to emit rays, and where M and q are both positive integers greater than or equal to 2; and N detectors 202 arranged at intervals along the circumferential direction c of the imaging channel 80, where the N detectors 202 are used to detect the rays emitted from the M distributed X-ray sources and penetrating the object 70 to be imaged, and where N is a positive integer greater than M. The detector 202 includes a first sub-detector 2021 and a second sub-detector 2022. The CT imaging system 200 includes a plurality of imaging components, the imaging component includes at least one distributed X-ray source and at least one first sub-detector 2021. In the same imaging component, the distributed X-ray source and the first sub-detector 2021 are arranged face-to-face in the radial direction r of the imaging channel 80. In the same imaging component, a plurality of target spots 2011 of the distributed X-ray source and at least a part of the first sub-detector 2021 are located in the same plane perpendicular to the first direction z, where the radial direction r is perpendicular to the first direction z. At least one second sub-detector 2022 is provided on at least one side of the at least one distributed X-ray source in the first direction z.

In this embodiment, the distributed X-ray source and the first sub-detector 2021 are not only arranged at intervals in the circumferential direction c, but also at least a part of the first sub-detector and at least one of a plurality of target spots are in a plane perpendicular to the imaging channel 80. Moreover, by providing the second sub-detector 2022 to collaborate with the first sub-detector 2021, it is possible to receive rays from the distributed X-ray source from different angles or positions, thereby expanding a detection range. Such three-dimensional spatial arrangement breaks through the existing design of circumferential arrangement, enabling the CT imaging system 200 to detect from a plurality of angles simultaneously, improving the accuracy and breadth of detection, and effectively solving the problems of limited angle scanning and data truncation.

Referring to FIG. 6, FIG. 7, FIG. 9A, FIG. 9B, FIG. 9C and FIG. 9D, the M distributed X-ray sources 210 to 2M0 are arranged along the circumferential direction c of the imaging channel 80. For example, the M distributed X-ray sources 210 to 2M0 are arranged at intervals along the circumferential direction c of the imaging channel 80.

Continuing to refer to FIG. 6, FIG. 7, FIG. 9A, FIG. 9B, FIG. 9C and FIG. 9D, the N1 first sub-detectors are arranged along the circumferential direction c of the imaging channel 80. For example, the N1 first sub-detectors are arranged at intervals along the circumferential direction c of the imaging channel 80.

In some exemplary embodiments, the M distributed X-ray sources 210 to 2M0 and the N1 first sub-detectors are alternately arranged along the circumferential direction c of the imaging channel 80. For example, the M distributed X-ray sources 210 to 2M0 are arranged at equal intervals along the circumferential direction c of the imaging channel 80, with one first sub-detector provided between two adjacent distributed X-ray sources. The N1 first sub-detectors are arranged at equal intervals along the circumferential direction c of the imaging channel 80, with one distributed X-ray source between two adjacent first sub-detectors.

In some exemplary embodiments, the M distributed X-ray sources and M first sub-detectors respectively enclose to form closed or approximately closed polygons. For example, the M distributed X-ray sources 210 to 2M0 are arranged at equal intervals along the circumferential direction c of the imaging channel 80, approximately located on a first circumference C1. The M first sub-detectors 20211 to 2021N1 are arranged at equal intervals along the circumferential direction c of the imaging channel 80, approximately located on a second circumference C2.

It should be understood that, FIG. 7, FIG. 9A, FIG. 9B, FIG. 9C and FIG. 9D show schematic plane views perpendicular to the first direction z, that is, paper surfaces on which FIG. 7, FIG. 9A, FIG. 9B, FIG. 9C and FIG. 9D are located are perpendicular to the first direction z. As shown in FIG. 7, FIG. 9A, FIG. 9B, FIG. 9C and FIG. 9D, in the plane perpendicular to the first direction z, the M distributed X-ray sources 210 to 2M0 and the N1 first sub-detectors enclose to form a closed or approximately closed polygon. For example, in FIG. 7, three distributed X-ray sources 210 to 230 and three first sub-detectors 20211 to 20213 enclose to form a hexagon; in FIG. 9A, two distributed X-ray sources 210 and 220 and two first sub-detectors 20211 and 20212 enclose to form a quadrilateral; in FIG. 9B, five distributed X-ray sources 210 to 250 and five first sub-detectors 20211 to 20215 enclose to form a decagon; in FIG. 9C, M distributed X-ray sources 210 to 2M0 and N1 first sub-detectors 20211 to 2021N1 enclose to form a (M+N1) side polygon; and in FIG. 9D, three distributed X-ray sources 210 to 230 and three first sub-detectors 20211 to 20213 respectively enclose to form triangles.

The arrangement of the X-ray source and detector determines a spatial resolution and a field of view of CT imaging. A geometric layout of this closed or approximately closed polygon optimizes the viewing angle of the imaging system, allowing each detector to receive signals from different angles, effectively increasing the number of projection data to cover the imaging area to the maximum extent.

Referring to FIG. 6, the distributed X-ray source may include a first side 211 and a second side 212 arranged opposite to each other in the first direction z, for example, the first side 211 is located downstream of the second side 212.

In embodiments of the present disclosure, for at least one distributed X-ray source, one or more second sub-detectors may be provided on at least one of the first side 211 and the second side 212 of the distributed X-ray source in the first direction z.

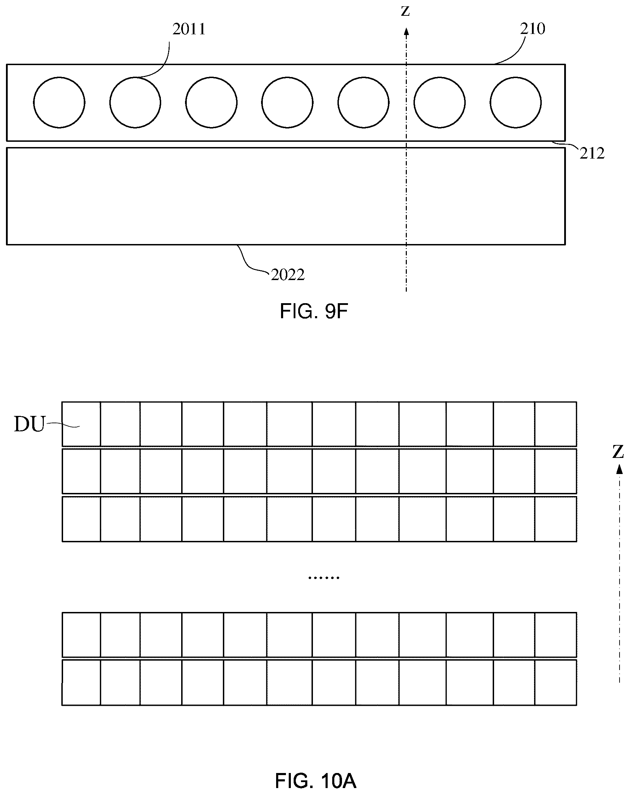

FIG. 9E shows a schematic diagram of an arrangement of the distributed X-ray source and the second sub-detector according to some embodiments of the present disclosure. FIG. 9F shows a schematic diagram of an arrangement of the distributed X-ray source and the second sub-detector according to some other embodiments of the present disclosure.

Referring to FIG. 6, in the first direction z, second sub-detectors 2022 are respectively provided on the first side 211 and the second side 212 of the distributed X-ray source.

Referring to FIG. 9E, in the first direction z, only the first side 211 of the distributed X-ray source is provided with a second sub-detector 2022.

Referring to FIG. 9F, in the first direction z, only the second side 212 of the distributed X-ray source is provided with a second sub-detector 2022.

In embodiments of the present disclosure, in the first direction z, each of M' distributed X-ray sources is provided with at least one second sub-detector 2022 on the first side 211; alternatively, in the first direction z, each of M' distributed X-ray sources is provided with at least one second sub-detector 2022 on the second side 212; alternatively, in the first direction z, each of M' distributed X-ray sources is provided with at least one second sub-detector 2022 on the first side 211 and at least one second sub-detector 2022 on the second side 212, where M' is greater than or equal to 1 and less than or equal to M.

In this embodiment, the second sub-detector 2022 is provided and a position of the second sub-detector is flexibly arranged to better cooperate with the first detector 2021. Rays emitted by the distributed X-ray sources may be received from different angles or positions, thereby expanding the detection range. Such three-dimensional spatial arrangement method breaks through the existing design of circumferential arrangement, enabling the imaging system to detect from multiple angles simultaneously, improving the accuracy and breadth of detection, and effectively solving the problems of limited angle scanning and data truncation.

By way of example, N2=2M, that is, the number of second sub-detectors N2 may be equal to twice the number of distributed X-ray sources, that is, one second sub-detector is provided on each of two sides of each distributed X-ray source. Through such arrangement, the detection range is expanded for each distributed X-ray source, better improving the accuracy and breadth of detection, thereby effectively solving the problems of limited angle scanning and data truncation.

In some exemplary embodiments of the present disclosure, the CT imaging system 200 includes a plurality of imaging components.

For example, in this embodiment, the imaging component may include at least one distributed X-ray source, at least one first sub-detector, and at least a part of at least one second sub-detector. In the same imaging component, the distributed X-ray source and the first sub-detector are arranged face-to-face in the radial direction r of the imaging channel 80, and the distributed X-ray source and at least a part of the second sub-detector are arranged face-to-face in the radial direction r of the imaging channel 80. For example, in FIG. 6 and FIG. 7, the first distributed X-ray source 210 and the 1st first sub-detector 20211 are arranged face-to-face in the radial direction r of the imaging channel 80, the first distributed X-ray source 210 and a part of the 3rd second sub-detector, and a part of the 4th second sub-detector are arranged face-to-face in the radial direction r of the imaging channel 80, the first distributed X-ray source 210 and a part of the 5th second sub-detector, and a part of the 6th second sub-detector are arranged face-to-face in the radial direction r of the imaging channel 80, and these face-to-face arranged elements form an imaging component; Similarly, the second distributed X-ray source 220 and parts of the detectors arranged face-to-face with the second distributed X-ray source form an imaging component, and the third distributed X-ray source 230 and parts of the detectors arranged face-to-face with the third distributed X-ray source form an imaging component.

In some exemplary embodiments of the present disclosure, in the same imaging component, a plurality of target spots of the distributed X-ray source and at least a part of the first sub-detector are located in a same plane perpendicular to the first direction z.

In some exemplary embodiments of the present disclosure, q target spots of at least one distributed X-ray source are arranged at intervals along a straight line. By way of example, referring to FIG. 6 and FIG. 7, the q target spots of the first distributed X-ray source 210 are arranged at intervals along the first straight line L1. The extension direction of the first straight line L1 may be perpendicular to both the first direction z and the radial direction r. In the same imaging component including the first distributed X-ray source 210 and the 1st first sub-detector 20211, the first straight line L1 and at least a part of the 1st first sub-detector 20211 (a part facing the first distributed X-ray source 210) are located in the same first plane P1 perpendicular to the first direction z.

In embodiments of the present disclosure, the CT imaging system adopts the source-detector coplanar structure. Specifically, a plurality of distributed X-ray sources and a part of a plurality of first sub-detectors are located in the same plane perpendicular to the first direction z. By adopting the distributed X-ray source and the source-detector coplanar design, more complete data are obtained at different angles, thereby reducing signal loss, obtaining finer image details, and allowing for faster scanning.

In some exemplary embodiments of the present disclosure, the target spots of the M distributed X-ray sources included in the CT imaging system are located in the same plane perpendicular to the first direction z. For example, referring to FIG. 6 and FIG. 7, the q target spots of the first distributed X-ray source 210, the q target spots of the second distributed X-ray source 220, and the q target spots of the third distributed X-ray source 230 are located in the same first plane P1 perpendicular to the first direction z.

In embodiments of the present disclosure, the overall source-detector arrangement of the CT imaging system adopts the source-detector coplanar structure. By adopting the distributed X-ray source and the overall source-detector coplanar design, a compact structure and a size reduction of the CT imaging system are achieved, while allowing for more complete data to be obtained at different angles, thereby reducing signal loss, obtaining finer image details, and allowing for faster scanning.

In embodiments of the present disclosure, the controller 50 may adjust the scanning mode of each target spot in the distributed X-ray source through several key parameters to achieve optimal image quality and imaging efficiency. For example, the controller 50 may control at least one of the following aspects of each target spot according to actual imaging requirements: an activation moment of each target spot, an emission range of each target spot, a beam emitting order of each target spot, an emission intensity of each target spot, or a beam emitting combination of the target spots.

In embodiments of the present disclosure, the M distributed X-ray sources may be arranged at intervals along the circumferential direction of the imaging channel, distributed at different angles in the imaging area, for irradiating the object to be imaged from a plurality of directions to obtain perspective images at different angles; the N1 first sub-detectors may also be arranged at intervals along the circumferential direction of the imaging channel, respectively facing the M distributed X-ray sources.

For example, as shown in FIG. 6, three distributed X-ray sources are distributed at different angles in a polygonal pattern in the imaging area. Such design may irradiate the object to be imaged with rays from a plurality of directions, thereby obtaining perspective images at different angles; opposite to the distributed X-ray sources, three detectors are arranged around the imaging area to receive the ray signals transmitted through the object to be imaged. The positional relationship between the detectors and the X-ray sources ensures that the system may obtain high-quality CT projection data.

As shown in FIG. 7, dashed lines marked in the figure represent farthest paths of the imaging light paths of the X-rays emitted from the distributed X-ray sources to reach the detectors on the opposite side after passing through the object to be imaged. For example, for the first distributed X-ray source 210 located on an upper side, a light path LP10 represents a light path of rays emitted from a leftmost first target spot and incident onto a detection unit of a second sub-detector on the opposite side. It should be noted that, due to a presence of a second sub-detector opposite to the first distributed X-ray source 210, the light path LP10 may irradiate the object to be imaged at a larger angle.

For example, a light path LP20 represents a light path of rays emitted from the q-th target spot located on a rightmost side and incident onto a detection unit of another second sub-detector on the opposite side. It should still be noted that, due to a presence of another second sub-detector opposite to the first distributed X-ray source 210, the light path LP20 may irradiate the object to be imaged at a larger angle.

The two light paths LP10 and LP20 intersect at a point Q10 in the plane P1 where the sources and the detectors are located. An area enclosed by the two light paths LP10 and LP20 and a detection surface of the detector is a first imaging area.

For the second distributed X-ray source 220 located on one side, a light path LP30 represents a light path of rays emitted from the first target spot located on a topmost side and incident onto a detection unit of the second sub-detector on the opposite side. A light path LP40 represents a path of rays emitted from the q-th target spot located at a lowermost side and incident onto a detection unit of another second sub-detector on the opposite side. The two light paths LP30 and LP40 intersect at a point Q20 in the plane P1 where the sources and detectors are located. An area enclosed by the two light paths LP30 and LP40 and a detection surface of the detector is a second imaging area.

For the third distributed X-ray source 230 located on the other side, a light path LP50 represents a light path of rays emitted from the first target spot located on a topmost side and incident onto a detection unit of a second sub-detector on the opposite side. A light path LP60 represents a light path of rays emitted from the q-th target spot located at a lowermost side and incident onto a detection unit of another second sub-detector on the opposite side. The two light paths LP50 and LP60 intersect at a point Q30 in the plane P1 where the sources and detectors are located. An area enclosed by the two light paths LP50 and LP60 and a detection surface of the detector is a third imaging area.

An overlapping area of the first imaging area, the second imaging area, and the third imaging area forms a polygonal area. For example, in the example of FIG. 7, a triangular area is formed, and a maximum inscribed circle of the polygonal area forms a valid imaging area S10.

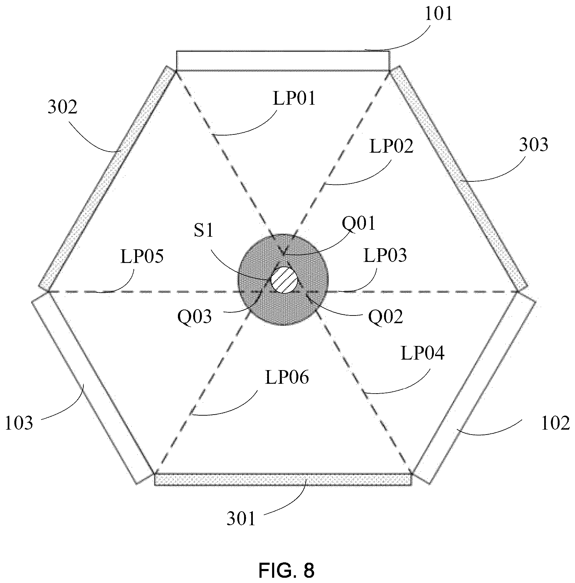

FIG. 8 is a schematic diagram of an imaging light path of a CT imaging system according to some other exemplary embodiments of the present disclosure. It should be noted that, the schematic imaging light path diagram shown in FIG. 8 may be considered as a comparative example of the schematic imaging light path diagram shown in FIG. 7. That is, the only difference between the CT imaging system targeted by FIG. 8 and the CT imaging system targeted by FIG. 7 (i.e., the CT imaging system shown in FIG. 6) is that: the second sub-detectors described above are not provided in the CT imaging system targeted by FIG. 8.

As shown in FIG. 8, dashed lines marked in the figure represent farthest paths of the imaging light paths of the X-rays emitted from the distributed X-ray sources to reach the detectors on the opposite side after passing through the object to be imaged. It should be noted that, the light paths of the distributed X-ray sources have been simplified in FIG. 8 for conciseness. Due to overlapping of light paths of rays emitted by the distributed X-ray sources, each light path shown by the dashed line in FIG. 8 may represent some overlapped ray light paths. Therefore, FIG. 8 may be regarded as a simplified version of the light paths in FIG. 3B.

For example, as shown in FIG. 8, for the first distributed X-ray source 101 located on an upper side, a light path LP01 represents a light path of rays emitted from the first target spot located on a leftmost side and incident onto a rightmost unit of the first detector 301; a light path LP02 represents a light path of rays emitted from the q-th target spot located on a rightmost side and incident onto a leftmost unit of the first detector 301. The two light paths LP01 and LP02 intersect at a point Q01 in the plane P1 where the sources and detectors are located. An area enclosed by the two light paths LP01 and LP02 and a detection surface of the first detector 301 is a first imaging area of the imaging component including the first distributed X-ray source 101 and the first detector 301. For the second distributed X-ray source 102 located on one side, a light path LP03 represents a light path of rays emitted from the first target spot located on a topmost side and incident onto a lowermost unit of the second detector 302, and a light path LP04 represents a light path of rays emitted from the q-th target spot located on a lowermost side and incident onto a topmost unit of the second detector 302. The two light paths LP03 and LP04 intersect at a point Q02 in the plane P1 where the sources and detectors are located. An area enclosed by the two light paths LP03 and LP04 and a detection surface of the second detector 302 is a second imaging area of the imaging component including the second distributed X-ray source 102 and the second detector 302. For the third distributed X-ray source 103 located on the other side, a light path LP05 represents a light path of rays emitted from the first target spot located on a topmost side and incident onto a lowermost unit of the third detector 303, and a light path LP06 represents a light path of rays emitted from the q-th target spot located on a lowermost side and incident onto a topmost unit of the third detector 303. The two light paths LP05 and LP06 intersect at a point Q03 in the plane P1 where the sources and detectors are located. An area enclosed by the two light paths LP05 and LP06 and a detection surface of the third detector 303 is a third imaging area of the imaging component including the third distributed X-ray source 103 and the third detector 303. An overlapping area of the first imaging area, the second imaging area, and the third imaging area forms a polygonal area, for example, in the example of FIG. 8, a triangular area is formed, and a maximum inscribed circle of the polygonal area forms a valid imaging area S1.

In FIG. 8, due to the absence of the second sub-detectors, only the region S1 in the object to be imaged may be completely covered by the detector, and there is a data truncation problem outside the region S1 (covered by only part of rays). However, in the system shown in FIG. 7, at least one side of the distributed X-ray source is provided with the second sub-detector, the angle range of the ray of each X-ray source that may be received by the detector is expanded. That is to say, by providing the second sub-detector, the valid imaging area may be expanded from S1 to S10 without changing positions and sizes of other components, thereby effectively reducing or even avoiding the data truncation problems.

It should be noted that, for a system with sources and detectors arranging in the circumferential direction, the rays intersect in a central area, forming a polygonal valid imaging area, and an intersection is a main imaging area. For the convenience of comparison and description, a tangent circle of the polygon is taken as the valid imaging area.

In embodiments of the present disclosure, the detector may be a single row detector, a multi-row detector, or an area detector, and suitable detector types may be selected in different application scenarios to optimize imaging efficiency and effect. Among them, the single row detector includes a plurality of detection units arranged along a straight line; the multi-row detector includes a plurality of rows of detector units, each row of detector units includes a plurality of detector units, forming a matrix or grid like structure; the area detector covers a large flat area, in which detection units are densely arranged to form a continuous detection surface.

In embodiments of the present disclosure, a flexible detector layout design suitable for the first sub-detector 2021 and the second sub-detector 2022 is also provided. That is to say, different arrangements may be designed to meet different imaging needs.

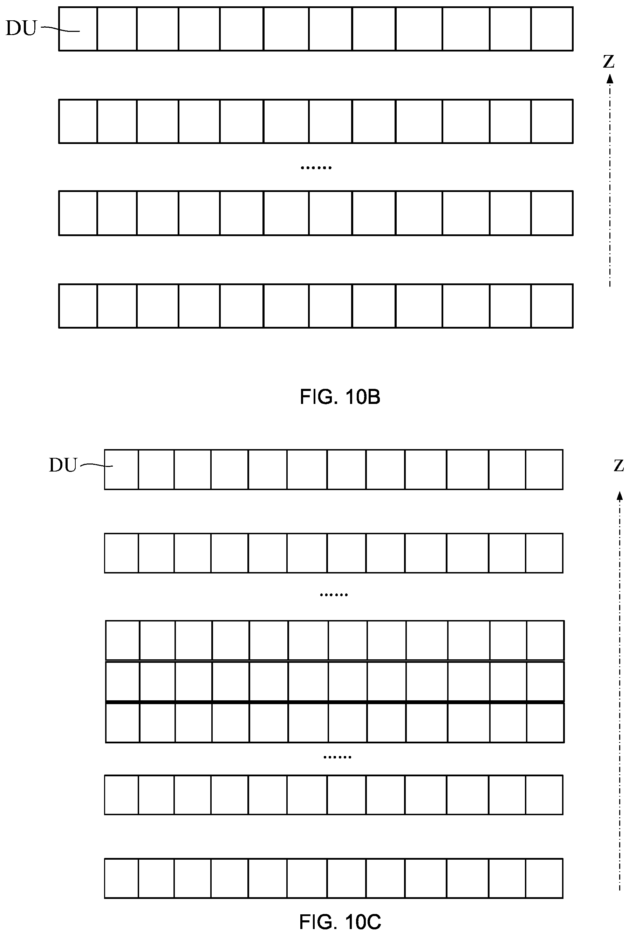

FIG. 10A shows a schematic diagram of a layout design of dense detector arrangement according to some embodiments of the present disclosure; FIG. 10B shows a schematic diagram of a layout design of sparse detector arrangement according to some other embodiments of the present disclosure; FIG. 10C shows a schematic diagram of a layout design of hybrid detector arrangement according to some other embodiments of the present disclosure.

Referring to FIG. 10A, FIG. 10B and FIG. 10C, for example, the first sub-detector 2021 may include a plurality of rows of detector units DU, which are densely arranged along the first direction z; alternatively, the first sub-detector 2021 may include a plurality of rows of detector units DU sparsely arranged along the first direction z; alternatively, the first sub-detector 2021 may include a plurality of rows of detector units DU, with at least two of the plurality of rows of detector units DU densely arranged in the first direction z in a middle area of the first sub-detector 2021, and at least two other of the plurality of rows of detector units DU sparsely arranged in the first direction z on two side areas of the first sub-detector 2021.

Similarly, the second sub-detector 2022 may include a plurality of rows of detector units DU densely arranged along the first direction z; alternatively, the second sub-detector 2022 may include a plurality of rows of detector units DU sparsely arranged along the first direction z; alternatively, the second sub-detector 2022 may include a plurality of rows of detector units DU, with at least two of the plurality of rows of detector units DU densely arranged in the first direction z in a middle area of the second sub-detector 2022, and at least two other of the plurality of rows of detector units DU sparsely arranged in the first direction z on two side areas of the second sub-detector 2022.

According to embodiments of the present disclosure, in a case of dense arrangement, the detectors may acquire more ray information, thereby enhancing the details of the image, which is particularly useful in imaging micro areas or situations requiring high-precision observation, such layout is suitable for high-resolution imaging, such as detecting micro structures or medical applications that require precise measurements; in a case of sparse arrangement, the costs and complexity may be reduced, or a sufficient image quality may be achieved without the need for high-resolution imaging, making it suitable for large-scale imaging or situations with high imaging speed requirements; for applications that require consideration of cost, imaging range, and detailed imaging of the central area, such as certain types of medical diagnosis or industrial examinations, the advantages of the above two configurations may be combined, the dense arrangement may be used in the middle area of the detector to ensure the high resolution of the imaging central area, while the sparse arrangement on the two sides may expand the imaging range while controlling the costs and the data processing complexity.

In embodiments of the present disclosure, the plurality of rows of detector units may further include a middle row of detector units, which is located at a middle position of the plurality of rows of detector units in the first direction z. In the same imaging component, the plurality of target spots of the distributed X-ray source and the middle row of detector units of the first sub-detector are located in a same plane perpendicular to the first direction. That is, the plurality of target spots of the distributed X-ray source and the middle row of detector units of the first sub-detector may be located in the same plane, ensuring a precise alignment between the X-ray source and detector, and improving the imaging quality and consistency.

According to embodiments of the present disclosure, the CT imaging system 200 may further include a plurality of source-detector components, the source-detector component includes a distributed X-ray source and at least one second sub-detector 2022. The plurality of source-detector components may be flexibly combined and arranged to meet different imaging needs. For example, the CT imaging systems may adapt to imaging requirements of different sizes, shapes, or resolutions by adding or reducing the source-detector components.

For example, an odd number of source-detector components may be provided, which may be three as shown in FIG. 6, or may be five, etc. By using an odd number of source-detector components, a symmetry and a balanced geometric center may be constructed, which helps optimize a distribution of rays and enable the CT imaging system to achieve more uniform coverage at different angles; meanwhile, an odd number of sources may evenly distribute rays, providing more consistent exposure in different directions of the object to be imaged, thereby reducing imaging errors caused by angular deviations.

In embodiments of the present disclosure, in the same source-detector component, an orthographic projection of the distributed X-ray source in the first direction z overlaps at least partially with an orthographic projection of at least one second sub-detector 2022 in the first direction z. That is to say, in the CT imaging system 200 according to embodiments of the present disclosure, the arrangement of the distributed X-ray source and the second sub-detector relative to the object to be imaged ensures that they have a good geometric alignment relationship in the imaging process to achieve a good detection effect.

Furthermore, in the same source-detector component, as shown in FIG. 6, a dimension d11 of the distributed X-ray source along the extension direction of the first straight line L1 and a dimension d12 of the second sub-detector 2022 along the extension direction of the first straight line L1 may be substantially equal to each other(dimensions of various distributed X-ray source are the same). Such design may ensure that the rays emitted by the X-ray source may be effectively captured by the detector along an entire length of the imaging channel.

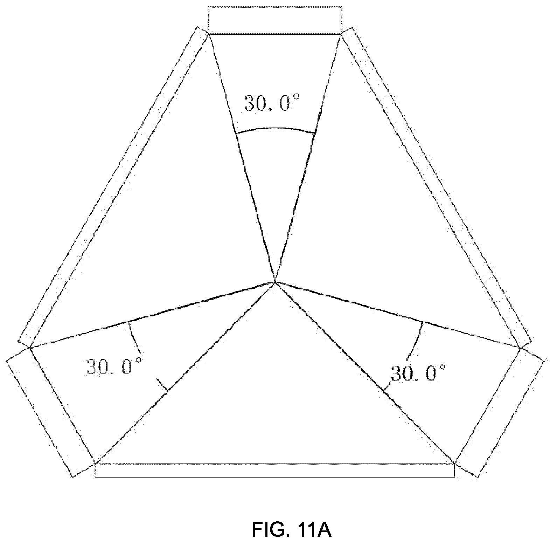

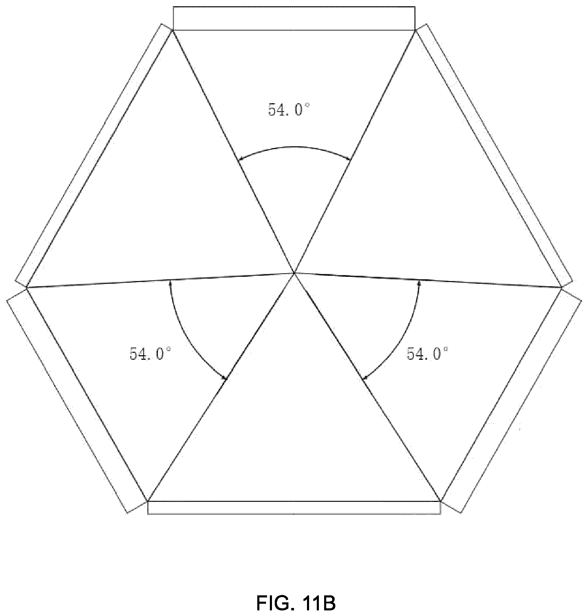

FIG. 11A shows a schematic diagram of a ray angular coverage range in a CT imaging system according to some embodiments of the present disclosure; FIG. 11B shows a schematic diagram of a ray angular coverage range in a CT imaging system according to some other embodiments of the present disclosure. It should be noted that, in the CT imaging systems illustrated in FIG. 11A and FIG. 11B, no second sub-detector is provided.

Taking the arrangement of providing three distributed X-ray sources as an example, as shown in FIG. 11A, a ray angular coverage range is represented by the line connecting a center point of the polygon and an edge of the distributed X-ray source. FIG. 11A shows that each X-ray source covers an angle of 30°, and a total scanning coverage range of 90° is provided; as shown in FIG. 11B, each X-ray source in FIG. 11B covers an angle of 54°, and a total scanning coverage range of 162° is provided.

Referring to FIG. 11A and FIG. 11B, the dimension of the X-ray source or a ratio of the dimension of the X-ray source to the dimension of the detector may be altered while keeping factors such as a relative position of the source and the detector, and a distance between the source and the detector constant, so that the ray angular coverage range of each distributed X-ray source may be changed, thereby a scanning coverage range of the CT imaging system may be changed. For example, by increasing the dimension of the distributed X-ray source (such as increasing the dimension of a single distributed X-ray source from the dimension shown in FIG. 11A to the dimension shown in FIG. 11B) or increasing the ratio of the dimension of the distributed X-ray source to the dimension of the detector, while keeping factors such as the relative position of the source and the detector and the distance between the source and the detector constant, the ray angular coverage range of a single distributed X-ray source may be increased, for example, from 30° to 54°. Correspondingly, the scanning coverage range of the CT imaging system may be increased from 90° to 162°.

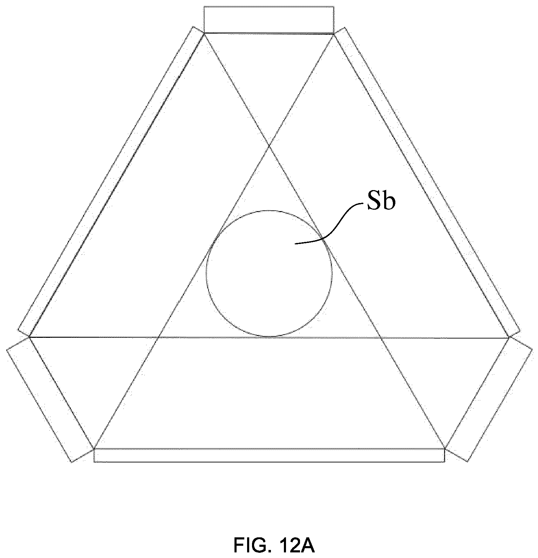

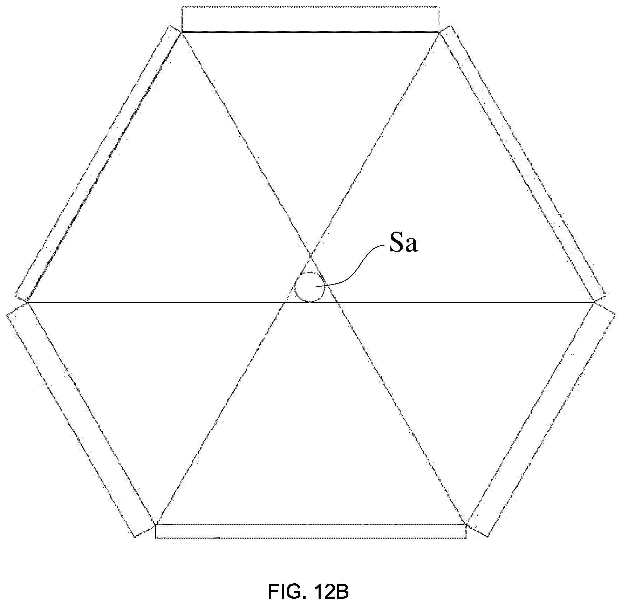

FIG. 12A shows a schematic diagram of a valid imaging area in a CT imaging system according to some embodiments of the present disclosure; FIG. 12B shows a schematic diagram of a valid imaging area in a CT imaging system according to some other embodiments of the present disclosure. It should be noted that, in the CT imaging systems illustrated in FIG. 12A and FIG. 12B, no second sub-detector is provided.

By referring to FIG. 12A and FIG. 12B, while keeping factors such as the relative position of the source and the detector and the distance between the source and the detector constant, the imaging area of each distributed X-ray source may be altered by changing the dimension of the X-ray source or the ratio of the dimension of the X-ray source to the dimension of the detector, thereby changing the valid imaging area of the CT imaging system. For example, by reducing the dimension of the distributed X-ray source (such as reducing the dimension of a single distributed X-ray source from the dimension shown in FIG. 12A to the dimension shown in FIG. 12B) or reducing the ratio of the dimension of the distributed X-ray source to the dimension of the detector, while keeping factors such as the relative position of the source and the detector and the distance between the source and the detector constant, the imaging area of a single distributed X-ray source may be increased. Correspondingly, the valid imaging area of the CT imaging system increases from Sa to Sb.

With reference to FIG. 11A, FIG. 11B, FIG. 12A and FIG. 12B, reducing the dimension of the distributed X-ray source or decreasing the ratio of the dimension of the distributed X-ray source to the dimension of the detector while keeping factors such as the relative position of the source and the detector and the distance between the source and the detector constant may increase the valid imaging area of the CT imaging system, but the scanning coverage range of the CT imaging system may be reduced; increasing the dimension of the distributed X-ray source or increasing the ratio of the dimension of the distributed X-ray source to the dimension of the detector may increase the scanning coverage range of the CT imaging system, but the valid imaging area of the CT imaging system may be reduced.

In embodiments of the present disclosure, by arranging the second sub-detector on a side of the X-ray source, it is possible to achieve an increase in the valid imaging area and an increase in the scanning coverage range, that is, the valid imaging area and the scanning coverage range may both be considered, thereby effectively improving the imaging quality.

Referring back to FIG. 6, FIG. 7, FIG. 9A, FIG. 9B, FIG. 9C, FIG. 9D, FIG. 9E and FIG. 9F, in embodiments of the present disclosure, by arranging the second sub-detector on a side of the X-ray source, the ray angular coverage range of the M distributed X-ray sources may reach a range of 90° to 120°; alternatively, the ray angular coverage range of the M distributed X-ray sources may reach a range of 120° to 150°; alternatively, the ray angular coverage range of the M distributed X-ray sources may reach a range of 140° to 160°.

In embodiments of the present disclosure, in the same imaging component, the distributed X-ray source has a first dimension a1 along the extension direction of the first straight line L1, the first sub-detector 2021 has a second dimension a2 along the direction parallel to the extension direction of the first straight line L1, and the first dimension a1 is smaller than the second dimension a2. The valid coverage range of the detector is greater than a length of a target spot line array, thereby facilitating a complete reception of ray beams emitted by each target spot by the detector and increasing the valid imaging area.

Through different methods for processing moving and stationary objects, embodiments of the present disclosure further provide a method for optimizing detector dimension so as to ensure a complete image data acquisition under various imaging conditions. For a moving object, a movement distance of the object and a ray amplification ratio needs to be consider by the detector to ensure coverage of all possible motion ranges; for a stationary object, the detector needs to ensure a coverage of an area of interest.

Specifically, for a moving object, a minimum dimension required for the detector may be calculated according to a movement speed of the object to be imaged and the ray geometric relationship. The dimension of the first sub-detector 2021 in the first direction z satisfies the following equation:

$$LD = d*p \quad (1)$$

where LD is the dimension of the first sub-detector in the first direction, d is a movement distance of the object to be imaged in the first direction within one exposure period, and p is the ray amplification ratio. The ray amplification ratio is defined as a ratio of a dimension of an image on the detector to an actual dimension of the object, describing a degree to which the image is amplified in the imaging process due to the different distances between the object and the detector.

For a stationary object, the dimension of the first sub-detector 2021 in the first direction z may be greater than or equal to the dimension of the area of interest of the object to be imaged in the first direction z, thereby ensuring that the detector may fully cover the area to be imaged, so as to obtain the complete projection data and avoid loss of the image information.

In embodiments of the present disclosure, as shown in FIG. 7, FIG. 9A, FIG. 9B and FIG. 9C, the M distributed X-ray sources 210 to 2M0 and the N1 first sub-detectors surround to form a closed or approximately closed polygon in the plane perpendicular to the first direction z. The valid imaging area S1 of the CT imaging system 200 may be located in the middle area of the polygon described above. It should be noted that, the term "middle area" here refers to an area where a geometric center of the polygon is located and/or an area where the geometric center is located that expands radially outward by a predetermined distance. Through such arrangement, the center of the imaging area coincides or almost coincides with the geometric center of the polygon, which may enable the rays to uniformly cover the area of interest of the object to be imaged, thereby avoiding loss of edge data.

In embodiments of the present disclosure, the conveying mechanism 40 is located in the middle area of the polygon described above. Such layout supports a collaboration between the valid imaging area of the CT imaging system and the conveying mechanism. The conveying mechanism may move the object to be imaged to the middle area of the polygon, so that the object to be imaged may always be located in an ideal imaging position in the imaging process, further improving the accuracy and consistency of imaging. Furthermore, the conveying mechanism may be controlled to ensure a stable and smooth movement of the object to be imaged in the imaging process, reducing or even avoiding issues such as image blur or deformation caused by positional deviation or irregular motion.

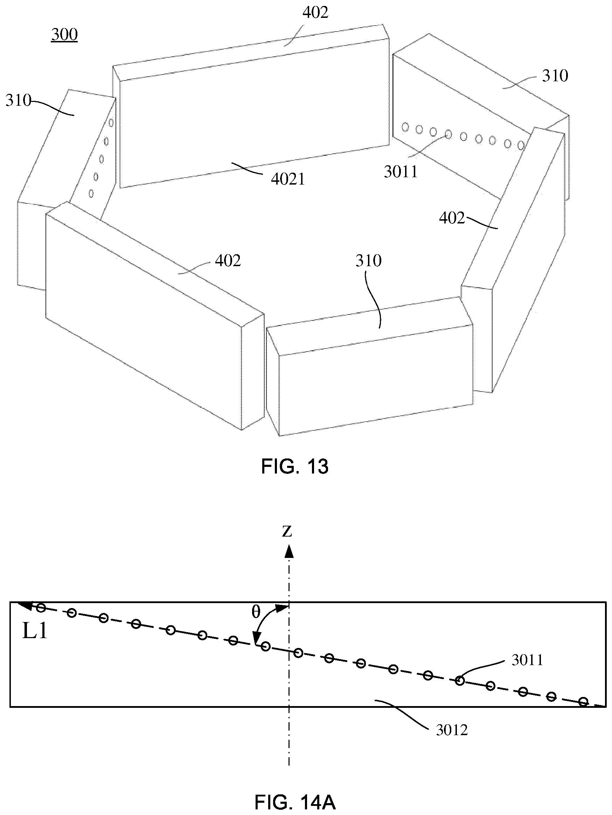

FIG. 13 is a schematic three-dimensional diagram of a CT imaging system according to some exemplary embodiments of the present disclosure, where target spots of the distributed X-ray source are obliquely arranged.

As shown in FIG. 13, the CT imaging system 300 may include: an imaging channel, where at least a part of the imaging channel extends along the first direction z, and the imaging channel is used to place the object to be imaged in the imaging process; M distributed X-ray sources 310 arranged at intervals along a circumferential direction of the imaging channel, where at least one distributed X-ray source 310 includes q target spots 3011, which are configured to be activated in a predetermined order to emit rays, and where M and q are both positive integers greater than or equal to 2; and N detectors 402 arranged at intervals along the circumferential direction of the imaging channel 80, for detecting the rays emitted from the M distributed X-ray sources 310 and penetrating the object to be imaged, where N is a positive integer greater than or equal to M.

FIG. 14A is a schematic diagram of a distributed X-ray source of a CT imaging system according to some exemplary embodiments of the present disclosure.

In some exemplary embodiments, the detector 402 includes a first sub-detector 4021, or the detector 402 consists of a first sub-detector 4021. That is to say, in this embodiment, as shown in FIG. 14A, the second sub-detector described above is not provided on an upper side or a lower side of the distributed X-ray source 310.

In this embodiment, the CT imaging system 300 includes a plurality of imaging components, the imaging component includes at least one distributed X-ray source 310 and at least one first sub-detector 4021. In the same imaging component, the distributed X-ray source 310 and the first sub-detector 4021 are arranged face-to-face in a radial direction r of the imaging channel. In the same imaging component, at least one target spot 3011 of the distributed X-ray source and at least a part of the first sub-detector 4021 are located in a same plane perpendicular to the first direction z, and the radial direction is perpendicular to the first direction z. In addition, the q target spots included in at least one of the M distributed X-ray sources are arranged obliquely relative to the first direction z.

The movement of objects in data acquisition may lead to inconsistency between consecutive image frames, which may result in motion artifacts. According to embodiments of the present disclosure, when the object to be imaged moves relative to the CT imaging system (e.g., the conveying mechanism drives the object to be imaged to move), the q target spots included in at least one of the M distributed X-ray sources are arranged obliquely relative to the first direction z, that is, there is a certain angle (refer to an angle θ in FIG. 14A) between an arrangement direction of the target spots and the first direction z, which reduces or even avoids motion artifacts, and thus improving the imaging quality.

In embodiments of the present disclosure, the CT imaging system 300 further includes a controller configured to control a beam emitting time interval of the plurality of target spots and control the movement speed of the object to be imaged.

As shown in FIG. 14A, the q target spots 3011 of the distributed X-ray source may be arranged at intervals along the first straight line L1. For example, the inclination angle θ of the first straight line L1 relative to the first direction z may satisfy the following equation:

$$\cos\theta = v*t/s \quad (2)$$

where s is a distance between two adjacent target spots among the q target spots, along the extension direction of the first straight line L1; v is a movement speed of the object to be imaged; and t is a beam emitting time interval of the distributed X-ray source.

As shown in FIG. 14A, the movement distance of the object 70 to be imaged along the first direction z within the beam emitting time interval t is v*t. The i-th target point and the (i+1)-th target point are two adjacent target spots, with a distance s along the extension direction of the first straight line L1. In the movement of the object 70 to be imaged along the first direction z, the i-th target point and the (i+1)-th target point emit beam in sequence according to the beam emitting time interval t. By setting s*cos$\theta$=v*t, the rays emitted by the i-th target point and the (i+1)-th target point scan a same cross-section of the object 70 to be imaged perpendicular to the first direction z. That is, when imaging the moving object 70 to be imaged, by designing an inclination angle of the target spots, the distance between the target spots, the beam emitting time interval, and the movement speed, the plurality of target spots and the object to be imaged are relatively stationary along the moving direction, thus a higher spatial resolution of a current scanning area may be obtained.

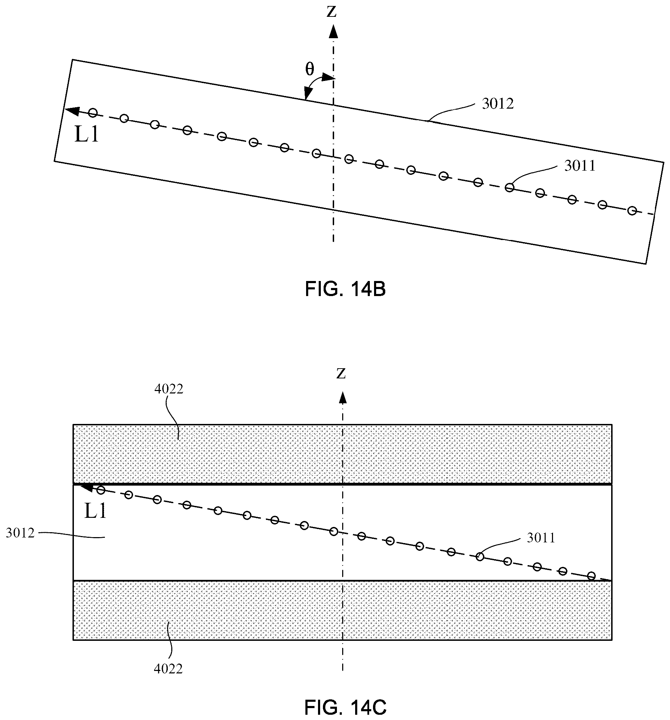

FIG. 14B is a schematic diagram of a distributed X-ray source of a CT imaging system according to some other exemplary embodiments of the present disclosure.

According to embodiments of the present disclosure, an inclination arrangement of the target spots relative to the first direction z may be achieved through different designs, thereby obtaining inclined target spots.

Referring to FIG. 14A and FIG. 14B, the distributed X-ray source 310 includes a X-ray source body 3012 and q target spots 3011 provided on the X-ray source body 3012.

As shown in FIG. 14A, the X-ray source body 3012 may be perpendicular to the first direction z, that is, two surfaces (an upper surface and a lower surface in FIG. 14A) of the X-ray source body 3012 along the first direction z are perpendicular to the first direction z. The q target spots 3011 provided on the X-ray source body 3012 are inclined relative to the two surfaces (the upper and lower surfaces in FIG. 14A) of the X-ray source body 3012 along the first direction z, so that the q target spots 3011 are obliquely arranged relative to the first direction z. In this embodiment, the plurality of target spots may be flexibly inclined relative to the X-ray source body 3012 to enhance a design freedom of the system.

As shown in FIG. 14B, the q target spots 3011 provided on the X-ray source body 3012 are arranged parallel to the two surfaces (upper and lower surfaces in FIG. 14B) of the X-ray source body 3012 along the first direction z. The X-ray source body 3012 may be arranged obliquely with respect to the first direction z, that is, the two surfaces (the upper and lower surfaces in FIG. 14B) of the X-ray source body 3012 along the first direction z are arranged obliquely relative to the first direction z. In this way, the q target spots 3011 are arranged obliquely relative to the first direction z. In this embodiment, an entire X-ray source body may be directly inclined, which is more convenient and easier to ensure an inclination consistency of all the target spots.

In embodiments of the present disclosure, the q target spots 3011 of the M distributed X-ray sources 310 may also be arranged obliquely relative to the first direction z. The inclined arrangement of the q target spots of each distributed X-ray source may better adapt to a geometric shape and a moving state of the object to be imaged.

In embodiments of the present disclosure, the q target spots 3011 of the M distributed X-ray sources 310 may also have equal inclination angles relative to the first direction z. The equal inclination angles of the q target spots in each distributed X-ray source may ensure a consistent coverage of the entire imaging area by the X-ray source.

In embodiments of the present disclosure, the q target spots 3011 of the M distributed X-ray sources 310 may have z-direction position coordinates in the first direction z. The z-direction position coordinates of the i-th target spots of the M distributed X-ray sources 310 may be equal to each other; or the z-direction position coordinates of the i-th target spots of the M distributed X-ray sources 310 may also be unequal to each other, where $1 \leq i \leq q$. That is, according to specific imaging requirements, the z-direction positions of the target spots may be adjusted to optimize the projection angle and distribution density of the rays.

Furthermore, the controller 50 may be used to control the target spots of the M distributed X-ray sources 310 with equal z-direction position coordinates to emit beams simultaneously. The equal z-direction position coordinates mean that these target spots are located on a same horizontal plane in the z-direction, or in other words, they are located at a same height in the CT scanning area. Therefore, such design ensures that the rays emitted by these target spots penetrate a same imaging cross-section, thereby ensuring that multi-angle data of the same cross-section may be acquired simultaneously, which helps to improve the details and accuracy of the cross-section image, and also reduces blurring and distortion caused by object movement.

In embodiments of the present disclosure, the detector 402 may further include a second sub-detector 4022. At least one second sub-detector 4022 is provided on at least one side of at least one distributed X-ray source 310 in the first direction z.

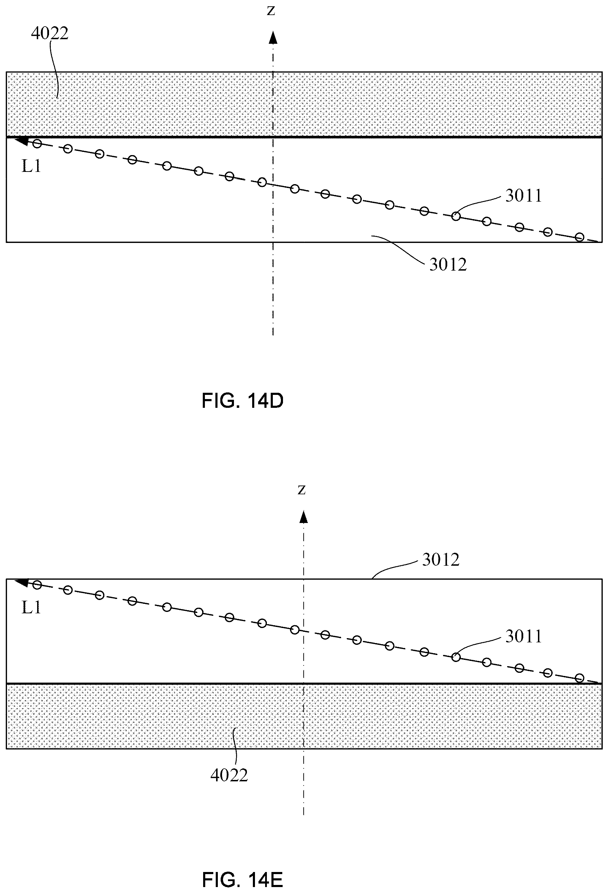

FIG. 14C, FIG. 14D and FIG. 14E show schematic diagrams of arrangements of a distributed X-ray source and a second sub-detector according to some embodiments of the present disclosure, where FIG. 14C shows that a second sub-detector is provided on each of two sides of a distributed X-ray source, FIG. 14D shows that a second sub-detector is provided on an upper side of a distributed X-ray source, and FIG. 14E shows that a second sub-detector is provided on a lower side of a distributed X-ray source.

Referring to FIG. 14C, in the first direction z, the second sub-detector 4022 is provided on each of the first side and the second side (upper and lower sides in FIG. 14C) of the distributed X-ray source.

Referring to FIG. 14D, in the first direction z, the second sub-detector 4022 is only provided on the first side 211 (the upper side in FIG. 14D) of the distributed X-ray source.

Referring to FIG. 14E, in the first direction z, the second sub-detector 4022 is only provided on the second side 212 (the lower side in FIG. 14E) of the distributed X-ray source.

In embodiments of the present disclosure, the plurality of target spots of at least one distributed X-ray source are arranged obliquely relative to the first direction z. Moreover, in the first direction z, each of M' distributed X-ray sources is provided with at least one second sub-detector 4022 on the first side 211; alternatively, in the first direction z, each of M' distributed X-ray sources is provided with at least one second sub-detector 4022 on the second side 212; alternatively, in the first direction z, each of M' distributed X-ray sources is provided with at least one second sub-detector 4022 on the first side 211 and at least one second sub-detector 4022 on the second side 212, where M' is greater than or equal to 1 and less than or equal to M.

In this embodiment, by obliquely arranging the plurality of target spots of the distributed X-ray source and providing the second sub-detector 4022, the position of the second sub-detector may be flexibly arranged to better collaborate with the first sub-detector 4021, the rays from the distributed X-ray source may be received from different angles or positions, thereby expanding the detection range. Such three-dimensional spatial arrangement method breaks through the existing design of circumferential arrangement, enabling the CT imaging system to detect from a plurality of angles simultaneously, improving the accuracy and breadth of detection, and effectively solving the problems of limited angle scanning and data truncation.

By way of example, N2=2M, that is, the number of second sub-detectors N2 may be equal to twice the number of distributed X-ray sources, which means that there is one second sub-detector on each of two sides of each distributed X-ray source. Through such arrangement, the detection range may be expanded for each distributed X-ray source, better improving the accuracy and breadth of detection, thereby effectively solving the problems of limited angle scanning and data truncation.

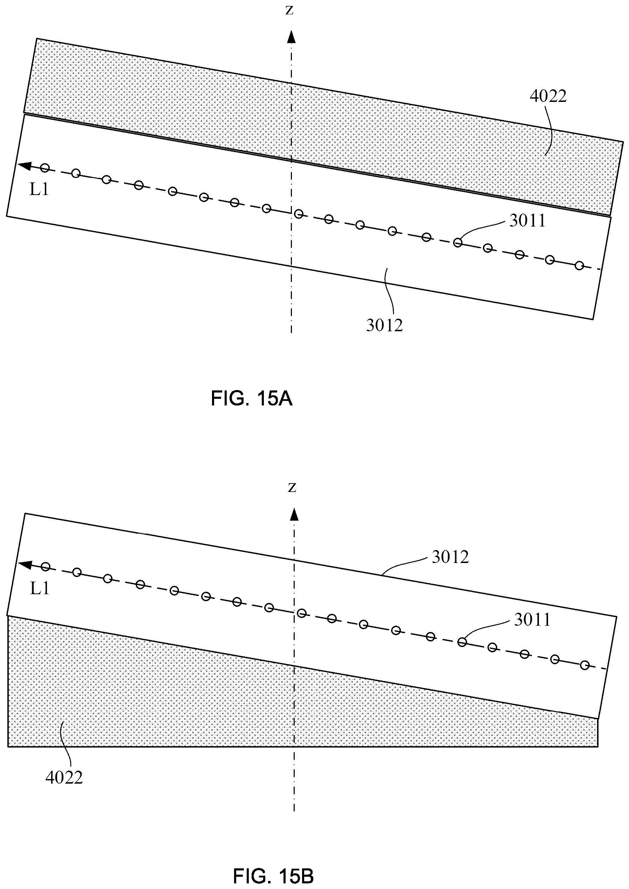

FIG. 15A shows a schematic diagram illustrating an arrangement of a second sub-detector according to some embodiments of the present disclosure. FIG. 15B shows a schematic diagram of an arrangement of a second sub-detector according to some other embodiments of the present disclosure.

Referring to FIG. 14C, FIG. 14D and FIG. 14E, in a case where the X-ray source body 3012 is perpendicular to the first direction z, the second sub-detector 4022 may be perpendicular to the first direction z. That is, the X-ray source body 3012 is parallel to the second sub-detector 4022.

Referring to FIG. 15A, in a case where the X-ray source body 3012 is arranged obliquely relative to the first direction z, the second sub-detector 4022 may be arranged parallel to the X-ray source body 3012, that is, the second sub-detector 4022 may be arranged obliquely relative to the first direction z. That is, two surfaces of the second sub-detector 4022 in the first direction z are obliquely arranged relative to the first direction z.

Referring to FIG. 15B, in a case where the X-ray source body 3012 is arranged obliquely relative to the first direction z, the second sub-detector 4022 may not be arranged parallel to the X-ray source body 3012, and at least one of the two surfaces of the second sub-detector 4022 in the first direction z is perpendicular to the first direction z.

According to embodiments of the present disclosure, a selection of inclined or vertical arrangement depends on a specific imaging goal, a nature of the object, and a desired imaging effect. The arrangement of the second sub-detector inclined at a certain angle relative to the first direction z may optimize the coverage of the detector on a specific area, especially in scenes that require capturing a motion trajectory or irregular shape of dynamic objects, but may require more complex calibration and algorithm tuning; the vertical arrangement is usually simple, easy to implement, and does not require complex angle calibration.

It should be noted that, a technical solution of the CT imaging system 300 provided in embodiments of the present disclosure may be combined with relevant features in the CT imaging system 200, and such combination is not limited by specific forms and methods, aiming to achieve better technical effects through various possible combinations. On this basis, an integration between the two systems may be flexibly adjusted and optimized according to actual application scenarios.

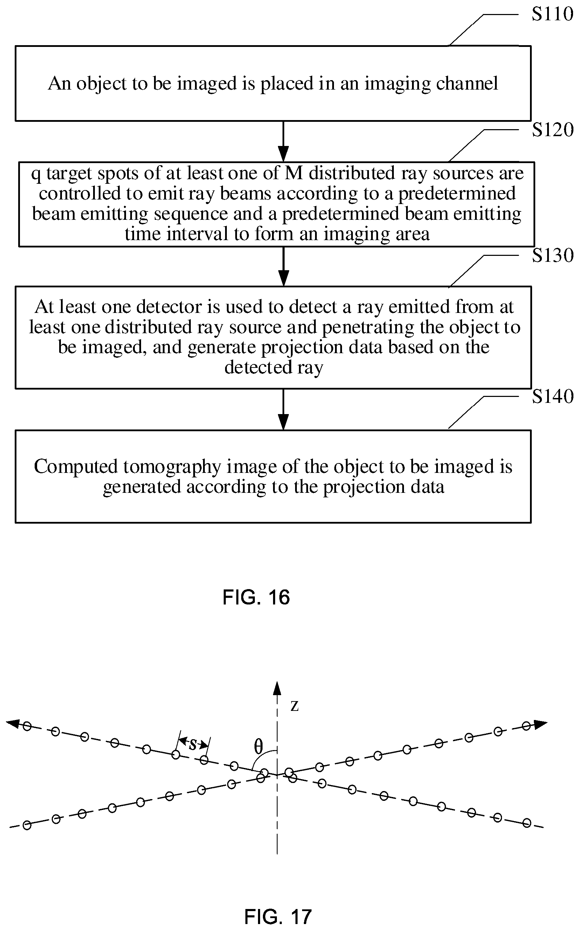

Embodiments of the present disclosure further provide an imaging method for a CT imaging system. FIG. 16 shows a flowchart of an imaging method for a CT imaging system according to some exemplary embodiments of the present disclosure.

As shown in FIG. 16, the imaging method may include steps S110 to S140. It should be noted that, the steps S110 to S140 are not limitations on an order of the imaging method. Without conflict, the imaging methods may be executed in parallel or in an order different from the one described herein.

In step S110, an object to be imaged is provided in an imaging channel.

In embodiments of the present disclosure, the object to be imaged may be controlled to move at a predetermined speed in the imaging channel.

In step S120, q target spots of at least one of M distributed X-ray sources are controlled to emit ray beams according to a predetermined beam emitting sequence and a predetermined beam emitting time interval to form an imaging area.

Furthermore, in order to reduce image artifacts caused by object motion, on the basis of the CT imaging system 300 in which the q target spots included in at least one of the M distributed X-ray sources are arranged obliquely relative to the first direction z, the control of the beam emitting time interval may be combined to ensure a continuity of image acquisition. Specifically, the beam emitting time interval may be determined based on an inclination angle of the q target spots relative to the first direction z.

In embodiments of the present disclosure, at the same moment, only one of the M distributed X-ray sources may generate X-rays. Alternatively, at the same moment, the plurality of target spots of one and same distributed X-ray source may generate X-rays. Alternatively, at the same moment, the target spots of the plurality of distributed X-ray sources at same positions may generate X-rays.

For example, when the object to be imaged is located in the imaging area, the k-th target spots of at least two of the M distributed X-ray sources may be controlled to emit beams simultaneously, where $1 \leq k \leq q$. The "emit beams simultaneously" refers to that in the imaging process, the emission of the ray beams may be precisely controlled in milliseconds or less, thereby controlling the target spots of at least two or more of the M distributed X-ray sources to emit ray beams at almost the same moment. The simultaneous beam emitting may obtain a large amount of data in a very short duration, significantly reducing an imaging duration.

FIG. 17 shows a schematic diagram of a projection plane of simultaneous beam emitting of a plurality of target spots in a CT imaging system according to some exemplary embodiments of the present disclosure.

As shown in FIG. 17, when the object to be imaged is located in the imaging area, the plurality of target spots may be controlled to emit beams in the following order: in a case where j values from 1 to q-1 in sequence, the j-th target spots of the M distributed X-ray sources emit beams simultaneously; then, the (j+1)-th target spots of the M distributed X-ray sources emit beams simultaneously. In the first direction z, the (j+1)-th target spot is located downstream of the j-th target spot.

For example, the order of beam emitting may be: the first target spots of all distributed X-ray sources, the second target spots of all distributed X-ray sources, ..., and so on. The first and second are only used to represent a positional relationship among X-ray sources or target spots. The beam emitting time interval may be determined based on the following equation:

$$t=s*cos\theta/v \quad (3)$$

where t is the beam emitting time interval, s is the distance between two adjacent target spots among the q target spots, v is the moving speed, and $\theta$ is the inclination angle of the q target spots relative to the first direction.

In embodiments of the present disclosure, when the object to be imaged is located in the imaging area, it is also possible to control the k-th target spots of at least two of the M distributed X-ray sources for a time-division beam emitting. The "time-division beam emitting" corresponds to "emit beam simultaneously", which refers to that in the imaging process, rays from different distributed X-ray sources may be emitted in sequence according to a predetermined beam emitting sequence and a predetermined beam emitting time interval, thereby separating the emitted beams in a time dimension. The time-division beam emitting may reduce a mutual interference between different ray beams, reduce signal overlap and noise, thereby improving the clarity and contrast of imaging.

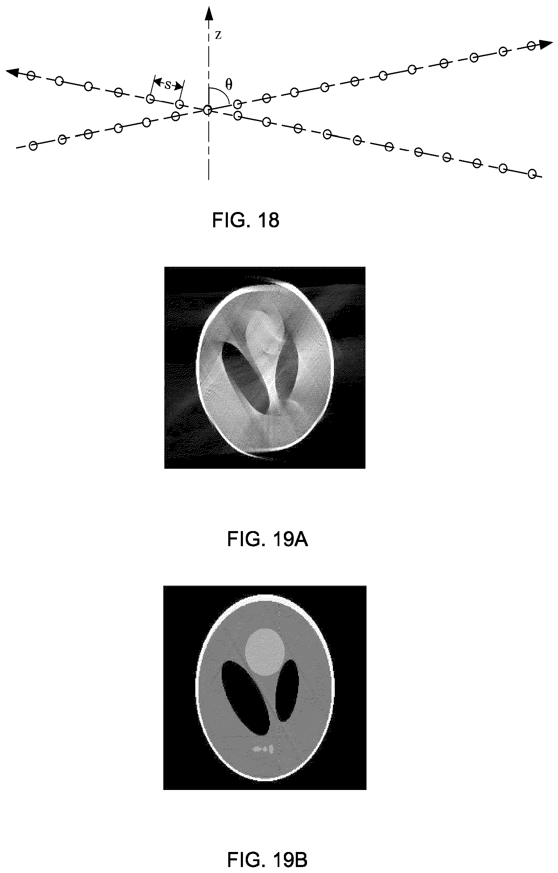

FIG. 18 shows a schematic diagram of a projection surface of time-division beam emitting of a plurality of target spots in a CT imaging system according to some exemplary embodiments of the present disclosure.

As shown in FIG. 18, when the object to be imaged is located in the imaging area, the plurality of target spots may be controlled to emit beams in the following order: in a case where j values from 1 to q-1 in sequence, the j-th target spots of the M distributed X-ray sources sequentially emit beams in a time-division manner; then, the (j+1)-th target spots of the M distributed X-ray sources sequentially emit beams in a time-division manner, where in the first direction, the (j+1)-th target spot is located downstream of the j-th target spot.

For example, the beam emitting order may be: the first target spot of the first distributed X-ray source, the first target spot of the second distributed X-ray source, ..., the first target spot of the N-th distributed X-ray source, the second target spot of the first distributed X-ray source, the second target spot of the second distributed X-ray source, ..., the second target spot of the N-th distributed X-ray source, and so on. The beam emitting time interval may be determined based on the following equation,

$$t=s*cos\theta/(M*v) \quad (4)$$

where t is the beam emitting time interval, s is the distance between two adjacent target spots among the q target spots, v is the moving speed, and $\theta$ is the inclination angle of the q target spots relative to the first direction.

In embodiments of the present disclosure, the beam emitting time interval satisfies: in the imaging process, the q target spots are in a relatively stationary state with the object to be imaged along the first direction, so that a higher spatial resolution of the current scanning area may be obtained.

By controlling the inclination of the X-ray source, a plurality of ray beams may scan the same cross-section during motion of an object. In this way, although a position of the object in space changes, the imaging process captures a relatively "stationary" perspective since the rays are always aligned with a specific part of the object. Therefore, such relatively stationary state ensures that the captured image does not experience a positional displacement due to the movement of the object in the imaging process. When the rays always irradiate on a same interface or path of the object, the continuity of image acquisition is maintained, greatly reducing the artifacts caused by motion.

In embodiments of the present disclosure, the beam emitting time interval of the CT imaging system may reach a microsecond level; a single acquisition duration of the detector may be in a range of 30 to 150 microseconds; and an imaging resolution of the imaging method may be in a range of 1 to 30 milliseconds.

In step S130, at least one detector is used to detect a ray emitted from at least one distributed X-ray source and penetrating the object to be imaged, and generate projection data based on the detected ray.

In embodiments of the present disclosure, when the ray penetrates the object to be imaged, tissues or substances of different densities and compositions may have different effects on absorption and scattering of the ray, which will be converted into projection data. These data are two-dimensional representations of an internal structure of the object, including information of viewing the imaged object from different angles.

In step 140, a computed tomography image of the object to be imaged is generated according to the projection data. Algorithms such as back projection and iterative reconstruction may be used to convert the two-dimensional projection data into three-dimensional images.

In embodiments of the present disclosure, the precise timing control and data acquisition of the imaging process are achieved by using the predetermined beam emitting sequence and the predetermined beam emitting time interval determined based on the inclination angle of the target spots relative to the first direction. The inclination arrangement of target spots and the precise beam emitting time interval may provide richer angle data, increase data redundancy, and help with subsequent image reconstruction and artifact reduction. The imaging method provided by the present disclosure not only significantly improves the imaging accuracy and quality, optimizes a synchronization of beam emitting and data acquisition in dynamic imaging, but also effectively reduces image distortion and artifacts caused by object movement.

FIG. 19A is an image obtained using an imaging system and an imaging method in the related art. FIG. 19B is an image obtained using the CT imaging system and the imaging method provided in embodiments of the present disclosure. Referring to FIG. 19A and FIG. 19B, in embodiments of the present disclosure, in the imaging process, the q target spots are in a relatively stationary state with the object to be imaged along the first direction, which effectively avoids motion artifacts and obtains high-quality images.

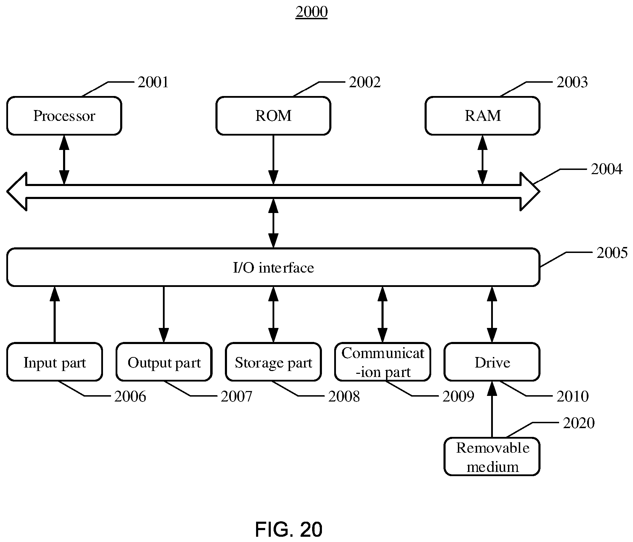

FIG. 20 schematically shows a block diagram of an electronic apparatus suitable for implementing the imaging method according to embodiments of the present disclosure. The electronic apparatus shown in FIG. 20 is only an example and should not impose any limitations on functionalities and scopes of use of embodiments of the present disclosure.

As shown in FIG. 20, an electronic apparatus 2000 according to embodiments of the present disclosure includes a processor 2001 that may perform various appropriate operations and processing based on programs stored in a read-only memory (ROM) 2002 or programs loaded from a storage part 2008 to a random access memory (RAM) 2003. The processor 2001 may include, for example, a general-purpose microprocessor (such as a CPU), an instruction set processor, and/or related chipsets and/or dedicated microprocessors (such as application specific integrated circuits (ASICs)), and so on. The processor 2001 may also include an onboard memory for caching purposes. The processor 2001 may include a single processing unit or a plurality of processing units for executing different operations of the method flow according to embodiments of the present disclosure.

Various programs and data required for operating the electronic apparatus 2000 are stored in the RAM 2003. The processor 2001, the ROM 2002, and the RAM 2003 are connected to each other through a bus 2004. The processor 2001 performs various operations of the method flow according to embodiments of the present disclosure by executing programs in the ROM 2002 and/or the RAM 2003. It should be noted that, the programs may also be stored in one or more memories other than ROM 2002 and RAM 2003. The processor 2001 may also perform various operations of the method flow according to embodiments of the present disclosure by executing the programs stored in the one or more memories.

According to embodiments of the present disclosure, the electronic apparatus 2000 may further include an input/output (I/O) interface 2005, which is also connected to the bus 2004. The electronic apparatus 2000 may also include one or more of the following components connected to the input/output (I/O) interface 2005: an input part 2006 including a keyboard, a mouse, etc.; an output part 2007 including cathode ray tubes (CrT), liquid crystal displays (LCD), and speakers; a storage part 2008 including a hard disk; and a communication part 2009 including network interface cards such as a LAN card, a modem, etc. The communication part 2009 performs communication processing via a network such as the Internet. A drive 2010 is also connected to the input/output (I/O) interface 2005 as needed. A removable medium 2020, such as a magnetic disk, an optical disk, a magneto-optical disk, a semiconductor memory, etc., is installed on drive 2010 as needed, so that computer programs read from the removable medium may be installed to the storage part 2008 as needed.

According to embodiments of the present disclosure, the method flow according to embodiments of the present disclosure may be implemented as a computer software program. For example, embodiments of the present disclosure include a computer program product, including a computer program carried on a computer-readable storage medium, the computer program includes program codes for executing the method shown in the flowchart. In such embodiments, the computer program may be downloaded and installed from the network through the communication part 2009, and/or installed from the removable medium 2020. When the computer program is executed by the processor 2001, it performs the above-described functions defined in the system of embodiments of the present disclosure. According to embodiments of the present disclosure, the system, apparatus, device, modules, units, etc. described above may be implemented through a computer program module.

The present disclosure further provides a computer-readable storage medium, which may be included in the apparatus/device /system described in the above embodiments; it may also exist independently without being assembled into the apparatus/device/system. The above-described computer-readable storage medium carries one or more programs, and the method according to embodiments of the present disclosure is implemented when the one or more programs are executed.

According to embodiments of the present disclosure, the computer-readable storage medium may be a non-volatile computer-readable storage medium, for example, including but not be limited to: a portable computer disk, a hard drive, a random access memory (RAM), a read-only memory (ROM), an erasable programmable read-only memory (EQROM or flash memory), a portable compact disk read-only memory (CD-ROM), an optical storage device, a magnetic storage device, or any suitable combination thereof. In the present disclosure, the computer-readable storage medium may be any tangible medium including or storing programs that may be used by an instruction execution system, apparatus, or device or in combination with the instruction execution system, apparatus, or device.

For example, according to embodiments of the present disclosure, the computer-readable storage medium may include one or more memories other than ROM 2002 and/or RAM 2003 described above.

Embodiments of the present disclosure further include a computer program product including a computer program including program codes for executing the method provided by embodiments of the present disclosure. When the computer program product is run on the electronic apparatus, the program codes are used to enable the electronic apparatus to implement the method provided by embodiments of the present disclosure.

When the computer program is executed by the processor 2001, it performs the above-described functions defined in the system/device of embodiments of the present disclosure. According to embodiments of the present disclosure, the system, device, modules, units, etc. described above may be implemented through a computer program module.

In an embodiment, the computer program may rely on a tangible storage medium such as an optical storage device, a magnetic storage device, etc. In another embodiment, the computer program may also be transmitted and distributed in a form of signals over network media, and downloaded and installed through the communication part 2009, and/or installed from the removable medium 2020. The program codes included in the computer program may be transmitted via any suitable network medium, including but not be limited to a wireless connection, a wired connection, etc., or any suitable combination thereof.

According to embodiments of the present disclosure, program codes for executing the computer program provided by embodiments of the present disclosure may be written in any combination of one or more programming languages. Specifically, these computer programs may be implemented using high-level procedural and/or object-oriented programming languages, and/or assembly/machine languages. The programming languages include but are not limited to programming languages such as Java, C++, qython, "C" language, or the like. The program codes may be executed entirely on a user computing apparatus, partially on a user apparatus, partially on a remote computing apparatus, or entirely on a remote computing apparatus or server. In cases involving a remote computing apparatus, the remote computing apparatus may be connected to the user computing apparatus through any type of network, including local area networks (LANs) or wide area networks (WANs), or may be connected to an external computing apparatus (such as using internet service providers for internet connection).

The flowchart and block diagram in the accompanying drawings illustrate possible implementation architectures, functions, and operations of the system, method, and computer program product according to the various embodiments of the present disclosure. In this regard, each box in the flowchart or block diagram may represent a module, program segment, or a part of code that includes one or more executable instructions for implementing specified logical functions. It should also be noted that, in some alternative implementations, the functions marked in the boxes may occur in an order different from those marked in the drawings. For example, two consecutive boxes may actually be executed in parallel, and sometimes they may also be executed in a reverse order, depending on the functions involved. It should also be noted that, each box in the block diagram or flowchart, as well as combinations of boxes in the block diagram or flowchart, may be implemented using a dedicated hardware based system that perform specified functions or operations, or may be implemented using a combination of dedicated hardware and computer instructions. Those skilled in the art may understand that, the features described in the various embodiments of the present disclosure may be combined and/or incorporated in various ways, even if such combinations or incorporations are not explicitly described in the present disclosure. Specifically, the features described in the various embodiments of the present disclosure may be combined and/or incorporated in various ways without departing from the spirit and teachings of the present disclosure. All these combinations and/or incorporations fall within the scope of the present disclosure.

Embodiments of the present disclosure have been described above. However, these embodiments are for illustrative purposes only and not to limit the scope of the present disclosure. Although each embodiment has been described separately above, it does not mean that the measures in each embodiment cannot be effectively combined. Without departing from the scope of the present disclosure, those skilled in the art may make various substitutions and modifications, and all the substitutions and modifications fall within the scope of the present disclosure.

**Claims**

1. A CT imaging system (100, 200, 300), comprising:

    an imaging channel (80), at least a part of the imaging channel (80) extending along a first direction (z), wherein the imaging channel (80) is configured to place an object to be imaged (70) in an imaging process;
    M distributed X-ray sources arranged along a circumferential direction (c) of the imaging channel (80), wherein at least one of the M distributed X-ray sources comprises q target spots for emitting rays, where M and q are positive integers greater than or equal to 2; and
    N detectors arranged along the circumferential direction (c) of the imaging channel (80), wherein the N detectors are configured to detect rays emitted from the M distributed X-ray sources and penetrating the object to be imaged, where N is a positive integer greater than M,
    wherein the N detectors comprise first sub-detectors and second sub-detectors;
    wherein the CT imaging system (100, 200, 300) comprises a plurality of imaging components, each of the plurality of imaging components comprises at least one distributed X-ray source and at least one first sub-detector; in the same imaging component, the distributed X-ray source and the first sub-detector are arranged face-to-face in a radial direction (r) of the imaging channel (80), a plurality of target spots of the distributed X-ray source and at least a part of the first sub-detector are located in a same plane perpendicular to the first direction (z), and the radial direction (r) is perpendicular to the first direction (z); and
    wherein at least one second sub-detector is provided on at least one side of the at least one distributed X-ray source in the first direction (z).

2. The CT imaging system (100, 200, 300) according to claim 1, wherein the distributed X-ray source comprises a first side (211) and a second side (212) opposite to each other in the first direction (z), and the first side (211) is located downstream of the second side (212); and
    wherein in the first direction (z), each of M' distributed X-ray sources is provided with at least one second sub-detector on the first side (211); or in the first direction (z), each of the M' distributed X-ray sources is provided with at least one second sub-detector on the second side (212); or in the first direction (z), each of M' distributed X-ray sources is provided with at least one second sub-detector on the first side (211) and at least one second sub-detector on the second side (212), where M' is greater than or equal to 1 and less than or equal to M.

3. The CT imaging system (100, 200, 300) according to claim 1 or 2, wherein the CT imaging system (100, 200, 300) comprises a plurality of source-detector components, the source-detector component comprises the distributed X-ray source and at least one second sub-detector; in the same source-detector component, an orthographic projection of the distributed X-ray source in the first direction (z) overlaps at least partially with an orthographic projection of the at least one second sub-detector in the first direction (z).

4. The CT imaging system (100, 200, 300) according to claim 3, wherein in the same distributed X-ray source, the q target spots are arranged at intervals along a first straight line (L1).

5. The CT imaging system (100, 200, 300) according to claim 4, wherein in the same source-detector component, a dimension of the distributed X-ray source along an extension direction of the first straight line (L1) is equal to a dimension of the second sub-detector along the extension direction of the first straight line (L1).

**6.** The CT imaging system (100, 200, 300) according to any one of claims 1 to 2 and 4 to 5, wherein the M distributed X-ray sources are uniformly arranged at equal intervals along the circumferential direction (c) of the imaging channel (80); and/or
the CT imaging system (100, 200, 300) comprises M first sub-detectors uniformly arranged at equal intervals along the circumferential direction (c) of the imaging channel (80).

**7.** The CT imaging system (100, 200, 300) according to any one of claims 1 to 2 and 4 to 5, wherein a ray angular coverage range of the M distributed X-ray sources is between 90° and 120°; or

a ray angular coverage range of the M distributed X-ray sources is between 120° and 150°; or
a ray angular coverage range of the M distributed X-ray sources is between 140° and 160°.

**8.** The CT imaging system (100, 200, 300) according to any one of claims 1 to 2 and 4 to 5, wherein the CT imaging system (100, 200, 300) comprises M first sub-detectors, and the M distributed X-ray sources and the M first sub-detectors are alternately arranged along the circumferential direction (c) of the imaging channel (80).

**9.** The CT imaging system (100, 200, 300) according to any one of claims 1 to 2 and 4 to 5, wherein the CT imaging system (100, 200, 300) comprises M first sub-detectors; and along the circumferential direction (c) of the imaging channel (80), the M distributed X-ray sources and the M first sub-detectors respectively enclose to form closed or approximately closed polygons.

**10.** The CT imaging system (100, 200, 300) according to any one of claims 1 to 2 and 4 to 5, wherein the CT imaging system (100, 200, 300) comprises 2M second sub-detectors.

**11.** The CT imaging system (100, 200, 300) according to claim 7, wherein in the same imaging component, the q target spots of the distributed X-ray source are arranged at intervals along a first straight line (L1), the distributed X-ray source has a first dimension (a1) along an extension direction of the first straight line (L1), the first sub-detector has a second dimension (a2) in a direction parallel to the extension direction of the first straight line (L1), and the first dimension (a1) is smaller than the second dimension (a2); and
wherein the second dimension (a2) of the first sub-detector is greater than or equal to a dimension of an area of interest of the object to be imaged (70) along the direction parallel to the extension direction of the first straight line (L1).

**12.** The CT imaging system (100, 200, 300) according to claim 11, wherein the second dimension of the first sub-detector satisfies the following equation:

$$LD=d*p,$$

where LD is the second dimension of the first sub-detector, d is a movement distance of the object to be imaged in the first direction within an exposure period, and p is a ray amplification ratio.

**13.** The CT imaging system (100, 200, 300) according to any one of claims 1 to 2, 4 to 5 and 11, wherein the first sub-detector comprises a plurality of rows of detector units densely arranged along the first direction (z); or

the first sub-detector comprises a plurality of rows of detector units sparsely arranged along the first direction (z); or
the first sub-detector comprises a plurality of rows of detector units, at least two of the plurality of rows of detector units are densely arranged in the first direction (z) in a middle area of the first sub-detector; and at least two other of the plurality of rows of detector units are sparsely arranged in the first direction (z) on two side areas of the first sub-detector; and
wherein the plurality of rows of detector units comprise a middle row of detector units located at a middle position of the plurality of rows of detector units in the first direction (z); and
wherein in the same imaging component, a plurality of target spots of the distributed X-ray source and the middle row of detector units of the first sub-detector are located in a same plane perpendicular to the first direction (z).

**14.** The CT imaging system (100, 200, 300) according to any one of claims 1 to 2, 4 to 5 and 11, wherein the second sub-detector comprises a plurality of rows of detector units densely arranged along the first direction (z); or

the second sub-detector comprises a plurality of rows of detector units sparsely arranged along the first direction (z); or
the second sub-detector comprises a plurality of rows of detector units, at least two of the plurality of rows of detector units are densely arranged in the first direction (z) in a middle area of the second sub-detector, and at least two other of the plurality of rows of detector units are sparsely arranged in the first direction (z) on two side areas of the second sub-detector.

**15.** The CT imaging system (100, 200, 300) according to claim 1 or 2, wherein in a plane perpendicular to the first direction (z), the M distributed X-ray sources and the N detectors enclose to form a closed or approximately closed polygon;

wherein a scanning imaging area of the CT imaging system (100, 200, 300) is located in a middle area of the polygon; and
the CT imaging system (100, 200, 300) further comprises a conveying mechanism (40) located in the imaging channel (80), wherein the conveying mechanism (40) is located in the middle area of the polygon.

FIG. 1

FIG. 2

FIG. 3A

FIG. 3B

FIG. 4A

FIG. 4B

FIG. 4C

FIG. 5A

FIG. 5B

FIG. 6

FIG. 7

FIG. 8

FIG. 9A

FIG. 9B

FIG. 9C

FIG. 9D

FIG. 9E

FIG. 9F

FIG. 10A

DU

Z

FIG. 10B

DU

Z

FIG. 10C

FIG. 11A

FIG. 11B

FIG. 12A

FIG. 12B

FIG. 13

FIG. 14A

FIG. 14B

FIG. 14C

4022

z

L1

3011

3012

**FIG. 14D**

z

3012

L1

3011

4022

**FIG. 14E**

z

4022

L1

3011

3012

FIG. 15A

z

3012

L1

3011

4022

FIG. 15B

S110

An object to be imaged is placed in an imaging channel

S120

q target spots of at least one of M distributed ray sources are controlled to emit ray beams according to a predetermined beam emitting sequence and a predetermined beam emitting time interval to form an imaging area

S130

At least one detector is used to detect a ray emitted from at least one distributed ray source and penetrating the object to be imaged, and generate projection data based on the detected ray

S140

Computed tomography image of the object to be imaged is generated according to the projection data

FIG. 16

FIG. 17

FIG. 18

FIG. 19A

FIG. 19B

2000

FIG. 20

Europäisches
Patentamt
European
Patent Office
Office européen
des brevets

**EUROPEAN SEARCH REPORT**

Application Number

EP 25 20 2136

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/261930 A1 (WANG GE [US] ET AL) 29 August 2019 (2019-08-29) * paragraphs [0023] - [0032]; figures 1,2 * ----- | 1-15 | INV. G01N23/046 |
| A | CN 114 947 911 A (UNIV TSINGHUA; NUCTECH CO LTD) 30 August 2022 (2022-08-30) * abstract; figures 2,3 * ----- | 1-15 | |
| A | CN 118 216 940 A (UNIV TSINGHUA) 21 June 2024 (2024-06-21) * abstract; figures 5,7 * ----- | 1-15 | |
| | | | **TECHNICAL FIELDS SEARCHED (IPC)** |
| | | | G01N A61B |

The present search report has been drawn up for all claims

| Place of search | Date of completion of the search | Examiner |
|---|---|---|
| The Hague | 27 February 2026 | Gilow, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 4 756 417 A1

## ANNEX TO THE EUROPEAN SEARCH REPORT
## ON EUROPEAN PATENT APPLICATION NO.

EP 25 20 2136

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2026

| Patent document cited in search report | | Publication date | Patent family member(s) | | Publication date |
|---|---|---|---|---|---|
| US 2019261930 | A1 | 29-08-2019 | US | 2019261930 A1 | 29-08-2019 |
| | | | WO | 2018085824 A1 | 11-05-2018 |
| CN 114947911 | A | 30-08-2022 | CN | 114947911 A | 30-08-2022 |
| | | | EP | 4300083 A1 | 03-01-2024 |
| | | | WO | 2022179387 A1 | 01-09-2022 |
| CN 118216940 | A | 21-06-2024 | NONE | | |